# EUROPÄISCHE PATENTSCHRIFT

(11) **EP 1 843 758 B1**
(45) Veröffentlichungstag und Bekanntmachung des Hinweises auf die Patenterteilung: **24.09.2014**
(21) Anmeldenummer: 06706619.1
(22) Anmeldetag: 03.02.2006
(51) Int. Cl.: A23L 1/30, A61K 9/20, A61K 9/30, A61K 9/36, A61K 31/085, A61K 31/7034, A61K 36/15, A61K 36/481, A61K 36/482, A61K 36/708, A61P 13/02, A61P 13/08, A61P 35/00

(54) **VERWENDUNG EINER HYDROXYSTILBEN-HALTIGEN WIRKSTOFFKOMBINATION ZUR BEHANDLUNG VON PROSTATAKREBS UND/ODER VON LUTS**
USE OF A THERAPEUTIC COMBINATION THAT CONTAINS HYDROXYSTILBENES FOR TREATING PROSTATE CANCER AND/OR LUTS
UTILISATION D'UNE COMBINAISON THERAPEUTIQUE A BASE D'HYDROXYSTILBENES POUR LE TRAITEMENT DU CANCER DE LA PROSTATE ET/OU DE LUTS

(30) Priorität: 04.02.2005 DE 102005005268; 04.02.2005 DE 102005005270; 04.02.2005 DE 102005005271; 04.02.2005 DE 102005005273; 04.02.2005 DE 102005005274; 04.02.2005 DE 102005005275; 04.02.2005 DE 102005005276; 19.05.2005 DE 102005023164
(43) Veröffentlichungstag der Anmeldung: 17.10.2007
(60) Teilanmeldung: 12177163.8
(73) Patentinhaber: Heger, Peter, 76698 Ubstadt-Weiher (DE); Rettenberger, Reinhard, 73033 Göppingen (DE); Spaich, Carl-Friedrich, 73092 Heiningen (DE)
(72) Erfinder: Heger, Peter, 76698 Ubstadt-Weiher (DE); Rettenberger, Reinhard, 73033 Göppingen (DE); Spaich, Carl-Friedrich, 73092 Heiningen (DE)
(74) Vertreter: Reitstötter Kinzebach
(86) Internationale Anmeldenummer: PCT/EP2006/000951
(87) Internationale Veröffentlichungsnummer: WO 2006/082068

(56) Entgegenhaltungen:
- WO-A-03/039557
- FR-A- 2 835 185
- ANONYM: "Phytoestrogen hilft bei Beschwerden im Klimakterium" ÄRZTE ZEITUNG, [Online] 16. Mai 2000 (2000-05-16), XP002378130 Gefunden im Internet: URL:http://www.aerztezeitung.de/docs/2000/ 05/16/089a1403.asp?cat=> [gefunden am 2006-04-24]
- KAGEURA T ET AL: "Inhibitors from rhubarb on lipopolysaccharide-induced nitric oxide production in macrophages: Structural requirements of stilbenes for the activity" BIOORGANIC AND MEDICINAL CHEMISTRY 2001 UNITED KINGDOM, Bd. 9, Nr. 7, 2001, Seiten 1887-1893, XP002378711 ISSN: 0968-0896 in der Anmeldung erwähnt
- KO S K ET AL: "Effect of stilbene derivatives from Rheum undulatum on carrageenan-induced acute edema in rats" KOREAN JOURNAL OF PHARMACOGNOSY 2004 SOUTH KOREA, Bd. 35, Nr. 2, 2004, Seiten 171-174, XP009065748 ISSN: 0253-3073
- RYU S Y ET AL: "Antitumor activity of some phenolic components in plants" ARCHIVES OF PHARMACAL RESEARCH, NATL. FISHERIES UNIVERSITY, PUSAN, KR, Bd. 17, Nr. 1, Januar 1994 (1994-01), Seiten 42-44, XP002968965 ISSN: 0253-6269
- CHUN YOUNG JIN ET AL: "Mechanism-based inhibition of human cytochrome P450 1A1 by rhapontigenin" DRUG METABOLISM AND DISPOSITION, Bd. 29, Nr. 4 Part 1, April 2001 (2001-04), Seiten 389-393, XP002378712 ISSN: 0090-9556
- WAFFO-TEGUO PIERRE ET AL: "Potential cancer-chemopreventive activities of wine stilbenoids and flavans extracted from grape (Vitis vinifera) cell cultures" NUTRITION AND CANCER, Bd. 40, Nr. 2, 2001, Seiten 173-179, XP009065742 ISSN: 0163-5581
- AGGARWAL B B ET AL: "Role of resveratrol in prevention and therapy of cancer: Preclinical and clinical studies" ANTICANCER RESEARCH 2004 GREECE, Bd. 24, Nr. 5 A, 2004, Seiten 2783-2840, XP009065753 ISSN: 0250-7005 in der Anmeldung erwähnt

## Beschreibung

Die Erfindung betrifft die Verwendung Hydroxystilben-haltiger Wirkstoffe zur Prävention und/oder Behandlung von Erkrankungen, deren Entstehung und/oder Verlauf mit einem erhöhten Serum-IL-6 Spiegel einhergeht und/oder deren Entstehung und/oder Verkauf durch Gabe eines selektiven Östrogenrezeptor-β (ERβ)-Agonisten behandelbar ist. Insbesondere betrifft die Erfindung die Behandlung von Prostatakrebs und/oder LUTS (Lower Urinary Tract Symptoms).

### HINTERGRUND DER ERFINDUNG

### 1. Allgemein

Wichtige Schlüsselfaktoren bei neurovegetativen Erkrankungen, wie Depressionen und Migräne, bei chronisch-entzündlichen Erkrankungen, bei der Prostatahyperplasie sowie bei der Tumorgenese und Tumorprogression sind die Freisetzung proinflammatorischer oder prokanzerogener Zytokine, und die anschließende Aktivierung von NF-κB-vermittelter Genexpression gefolgt von einer Reihe von Second Messengem, welche zur Entstehung dieser Erkrankungen führen oder deren Progression fördern. Beispielsweise werden große Mengen an TNF-α, Interleukin-1 (IL-1) und IL-6 als potente proinflammatorische und prokanzerogene Zytokine von Makrophagen und vielen anderen Zellen produziert (Long & Gardam, CMAJ 2003; 168: 1153 - 1156; Dinarello, Curr. Op. Pharmacol. 2004; 4: 378 - 385).

Zusätzlich zur erhöhten Zytokinproduktion werden Störungen in der Produktion von Neurotransmittern wie z.B. Serotonin und Dopamin sowohl für Migräne und Depressionen als auch für das Tumorwachstum verschiedener Organe und Gewebe verantwortlich gemacht (Gilbert. Eur J Cancer Prev 1997; 6: 269-276; Spinelli. Clin Obstet Gynecol 2004; 47: 428-436; Amorino & Parsons. Crit Rev Eukaryot Gene Expr 2004; 14: 287-300; Dizeyi et al. Prostate 2004 ; 59: 328-336 ; Siddiqui et al. Oncol Rep 2005; 14: 1593-1597).

Ein wichtiger Regulator neurovegetativer Prozesse und Induktor antiinflammatorischer Mechanismen bei Menschen ist Östrogen. Es wurde insbesondere gezeigt, dass ein Östrogenmangel das Ansprechen von Zellen auf Zytokine fördert, indem die Zahl der Zytokinrezeptoren und Kofaktoren der Zytokinwirkung hochreguliert und somit die Effekte der Zytokinsteigerung weiter verstärkt werden. Im Gegensatz dazu wurde beobachtet, dass insbesondere die Serum-IL-6-Spiegel in postmenopausalen Frauen unter Hormonersatztherapie (HRT) niedriger sind (Pfeilschifter et al, Endocrine Rev. 2002; 23: 90-119). Somit ist Östrogen ein entscheidender negativer Regulator von IL-6, und dessen Herunterregulation wird gestört, wenn die Östrogenspiegel abnehmen. Zudem verbessern Östrogene den Neurotransmitter-Status durch Veränderungen der Hormone der Hypothalamus-Hypophasen-Achse.

Im Mittelpunkt zukünftiger Therapien, die sowohl die Zytokin- als auch die Neurotransmitterproduktion regulieren, stehen als wichtige Steuerungselemente die Östrogenrezeptoren-α (ERα) und -β (ERβ). Durch Bindung an diese Kernrezeptorproteine regulieren Östrogene die Genexpression und kontrollieren damit viele Prozesse im Rahmen der Entwicklung, Differenzierung und Homöostase in Säugern (Kuiper et al., Proc. Natl. Acad. Sci. USA, 1996; 93: 5925 - 5930). Beiden Rezeptoren weisen nur partiell strukturelle Ähnlichkeiten auf, und aufgrund beträchtlicher Unterschiede in der N-terminalen Domäne und in der Liganden-Bindungsdomäne sind deren unterschiedliche biologische Funktionen zu erklären (Kuiper et al., Proc. Natl. Acad. Sci. USA, 1996; 93: 5925 - 5930; Enmark et al., J. Clin. Endocrinol. Metab. 1997; 82: 4258 - 4265). Insbesondere die Funktion des ERß ist nur teilweise aufgeklärt.

### 2. Chronisch-entzündliche Erkrankungen und Migräne

Im Rahmen der Entwicklung selektiver Östrogenrezeptor-ß (ERß)-Agonisten in Tiermodellen wurde ein signifikanter vorteilhafter Effekt einer der untersuchten Verbindungen bei chronisch-entzündlichen Darmerkrankungen beobachtet (Harris et al., Endocrinol. 2003; 144: 4241 - 4249). Tägliche orale Dosen von 1 mg/kg des ERβ-Agonisten beseitigten chronischen Durchfall und intestinale Läsionen bei Ratten. Inflammatorische Infiltrate wurden verringert und die Integrität der Darmmukosa wurde wieder hergestellt. Der Schweregrad der Erkrankung wurde um 50-60% reduziert.

In Untersuchungen im Lewis Rattenmodell für Adjuvans-induzierte Arthritis wurde ebenfalls eine signifikante Verbesserung der Arthritis beobachtet (Harris et al., Endocrinol. 2003; 144: 4241-4249). Inflammatorische Zellinfiltrate wurden verringert, eine Fibroblastenhyperplasie wurde reduziert und die proliferative Pannus-Reaktion wurde eliminiert.

Eine Studie der gleichen Arbeitsgruppe (Harris et al. Hum. Reprod. 2005; 20: 936-941) in einem Endometriosemodell in Mäusen, denen humanes Endometrium implantiert wurde, zeigte eine komplette Remission der Läsionen durch den ERß Agonisten. Dies wurde vermutlich durch eine ERβ-vermittelte Aktivierung von Immunzellen, welche den ERß exprimieren (Curran et al., Cell. Immunol. 2001; 214: 12-20, Stygar et al., Mol. Hum. Reprod. 2001; 7: 881-886, Henderson et al., J. Clin. Endocrinol. Metab. 2003; 88: 440-449, Vegeto et al., J. Neurosci. 2001; 21: 1809-1818), verursacht, die dann durch Apoptose das entzündete Gewebe eliminierten.

Inflammatorische Zytokine und Entzündungsreaktionen sind auch die Ursache für Migräne. Zytokine stimulieren die induzierbare NO-Synthase (iNOS) in der Dura mater, einem schmerzsensitiven intrakranialen Gewebe, wodurch große Mengen an Stickoxid (NO) gebildet werden (Reuter et al., Ann Neurol 2002; 51: 507-516). Daher führt die Verabreichung eines NOS-Inhibitors bei zwei Drittel der Patienten während einer akuten Attacke zu einer signifikanten Reduktion der Kopfschmerzen. Zusätzlich wird der Spiegel der Zytokine Interleukin (IL)-1β und IL-6 sowie von reaktiven Sauerstoffspezies aufgrund von oxidativem Stress erhöht. Diese Zytokine potenzieren die iNOS Expression und somit die NO-Produktion (Reuter et al, Ann Neurol 2002; 51: 507-516).

Es konnte gezeigt werden, dass Östrogene mit der Bindung des NF-κB/RelA-Proteins an entsprechende Stellen im Promotor, beispielsweise von IL-6 und iNOS, wechselwirken und damit deren Genexpression unterbinden (Stein et al., Mol Cell Biol. 1995; 15: 4971-4979; Galien et al., Nucleic Acids Res. 1997; 25: 2424-2429; Wen et al., Brain Res. 2004; 1008: 147-154). Vor allem die Aktivierung des ERß scheint für die Unterdrückung der Entzündungsmediatorproduktion in Mikroglia-Zellen in bestimmten Bereichen des Gehirns verantwortlich zu sein (Baker et al., Endocrinol. 2004; 145: 5021-5032).

Damit könnte die Aktivierung des ERß durch ERß-selektive Liganden und die nachfolgende Hemmung der Zytokinproduktion ein entscheidender Mechanismus zur Hemmung von Prozessen bei chronisch-entzündlichen Erkrankungen und bei Migräne sein.

### 3. Depressionen und Ängstlichkeit

Auch die anxiolytischen und antidepressiven Effekte von Östrogen werden über den ER vermittelt. Eine Östrogen-Behandlung erhöht die Aktivität des dopaminergen und serotoninergen Systems und lindert psychische Symptome beim Menschen. Ovarektomierte ERß-Knockout-Mäuse zeigten erhöhte Ängstlichkeit sowie signifikant reduzierte Dopamin- und Serotonin-Spiegel im Vergleich zu den Wildtyp-Mäusen. Dagegen scheint eine erhöhte Ängstlichkeit über den ERα vermittelt zu sein (Imwalle et al. Lack of functional Estrogen receptor β influences anxiety behavior and serotonin content in female mice. Physiology & Behavior 2005; 84: 157-163).

Im Forced-Swimming Test, einem Modell zur Untersuchung der antidepressiven Wirkung von Arzneimitteln, zeigten ovarektomierte ERβ -k.o.-Mäuse eine erhöhte Immobilität, die auch durch eine Estradiol-Gabe nicht aufgehoben werden konnte. Damit wurde postuliert, dass Substanzen, die selektiv ERß aktivieren, Ängstlichkeit und Depressionen reduzieren können (Imwalle et al. 2005; Lund et al. Novel Actions of Estrogen Receptor Beta on Anxiety-Related Behaviors. Endocrinology 2005; 146: 797-807; Rocha et al. 17β-Estradiol-induced antidepressant-like effect in the forced swim test is absent in estrogen receptor-β knockout (BERKO) mice. Psychopharmacology 2005, 179:637-643). In der Tat konnte in einem Rattenmodell die Gabe von ERβ-selektiven Aktivatoren wie z.B. Diarylpropionitril (DPN) oder Coumestrol die Ängstlichkeit der Tiere drastisch reduzieren (Walf & Frye. ERβ-selective estrogen receptor modulators produce antianxiety behavior when administered systemically to ovariectomized rats. Neuropsychopharmacology 2005; 1-12).

Auch bei Depressionen spielen proinflammatorische Cytokine, insbesondere IL-6, eine wichtige Rolle (Miller & O'Callaghan: Depression, cytokines, and glial function. Metab Clin Exp 2005; 54: 33-38; Alesci et al.: Major depression is associated with significant diurnal elevations in plasma interleukin-6 levels, a shift of ist circadian rhythm, and loss of physiological complexity in ist secretion: clinical implications. J Clin Endocrinol Metab 2005; 90: 2522-2530; Motivala et al.: Inflammatory markers and sleep disturbande in major depression. Psychosom Med 2005; 67: 187-194; Anisman et al.: Cytokines as a precipitant of depressive illness: animal and human studies. Curr. Pharm. Des. 2005; 11: 963-972). In Tiermodellen wurde gezeigt, dass die Behandlung mit z.B. IL-6 krankhaftes Verhalten sowie Symptome von Depressionen auslöste, die nur durch chronische Behandlung mit Antidepressiva behandelt werden konnten (Anisman et al. 2005). Bei Patienten führt eine Immuntherapie mit IL-2 oder TNF-α zu starken depressiven Symptomen, welche auf die erhöhten IL-6-Spiegel zurückgeführt werden.

Interessanterweise wurde bei Patienten mit Depressionen festgestellt, dass die Behandlung mit Antidepressiva nicht zu einer Reduktion der Cytokin-Spiegel, insbesondere auch nicht von IL-6 führte (Anisman et al. 2005, a.a.O.). Dies bedeutet, dass Antidepressiva zwar die Symptome verringern oder maskieren, die Auslöser jedoch bestehen bleiben. Dies kann eine Ursache der häufigen Rückfälle bei der Therapie mit den üblicherweise eingesetzten Antidepressiva sein.

### 4. Tumorgenese und -progression

IL-6 ist außerdem ein wichtiger Schlüsselfaktor bei der Tumorgenese. Bei den verschiedensten Tumorerkrankungen konnten erhöhte IL-6-Plasmaspiegel gemessen werden, die nur durch Chemotherapie gesenkt werden konnten. Tumorzellen produzieren selbst IL-6, welches in einem autokrinen Mechanismus das Wachstum und die Migration der Zellen stimuliert. IL-6 wird daher für die Veränderungen der Proliferation und Enthemmung der Differenzierung in vielen malignen Tumoren (Brust, Prostata, Lungenlymphoma, Ovarien, Magen-Darmtrakt, Nieren) verantwortlich gemacht. Zudem scheint es auch für die Chemoresistenz von soliden und hämatopoietischen Tumoren verantwortlich zu sein (zur Übersicht siehe: Dijsselbloem et al.: Soy isoflavone phytopharmaceuticals in interleukin-6 affections. Multi-purpose nutraceuticals at the crossroad of hormone replacement, anti-cancer and anti-inflammatory therapy. Biochem Pharmacol. 2004; 68:1171-1185).

Insbesondere gilt IL-6 als Prognosemarker in Patienten mit hormonunabhängigen Tumoren wie z.B. dem Prostatakarzinom (Drachenberg et al.: Circulating levels of interleukin-6 in patients with hormone refractory prostate cancer. Prostate 1999; 41:127-133) oder dem Mammakarzinom (Bachelot et al.: Prognostic value of serum levels of interleukin 6 and of serum and plasma levels of vascular endothelial growth factor in hormone-refractory metastatic breast cancer patients. Brit. J. Cancer 2001; 88:1721-1726). Hohe IL-6 Serumspiegel korrelieren bei diesen Tumoren mit einer kürzeren Lebenserwartung.

Dies zeigt, dass eine Reduktion der IL-6-Spiegel entscheidend zur Verbesserung der Überlebenschancen und der Remissionsfreiheit sowohl von endokrin-abhängigen als auch -unabhängigen Tumoren beitragen kann. Die bisherige Therapie erfolgt mit den klassischen unspezifischen Chemotherapeutika, die bekanntermaßen durch massive Nebenwirkungen und Unverträglichkeiten gekennzeichnet ist (Lehrbuch Mutschler et al.: Arzneimittelwirkungen). Eine spezifische anti-IL-6-Tumortherapie mit einem monoklonalen Antikörper gegen IL-6 wurde zur Senkung der IL-6-Spiegel in mehreren klinischen Studien durchgeführt (Trikha et al.: Targeted anti-interleukin-6 monoclonal antibody therapy for cancer: a review of the rationale and clinical evidence. Clin Cancer Res. 2003; 9:4653-4665). Als Nachteil stellte sich jedoch heraus, dass beim Einsatz des murinen anti-IL-6-Antikörpers bei 20 % der Patienten Antikörper gegen den Fc-Teil des Maus- Antikörpers gebildet wurden und dieser dadurch sehr schnell eliminiert wurde. Dadurch war keine Hemmung der IL-6-Wirkung in vivo mehr zu erzielen.

Daher gilt es, Arzneistoffe zu finden, die auch nach einer länger andauernden Einnahme eine Reduktion der IL-6-Spiegel bewirken und so eine Tumorprogression oderremission verhindern, ohne Nebenwirkungen oder eine Chemoresistenz zu verursachen.

Wie schon erwähnt, wird die Expression von IL-6 durch Östrogen über eine ERabhängigen Mechanismus unterdrückt. Dies korreliert mit der Beobachtung, dass die IL-6-Konzentrationen deutlich höher in solchen Mammakarzinomen lagen, die ERnegativ und einer Tamoxifen- oder adjuvanten Chemotherapie nicht zugänglich waren (Jiang et al.: Reduction in serum IL-6 after vacination of breast cancer patients with tumour-associated antigens is related to estrogen receptor status. Cytokine 2000; 12:458-465).

Auf der Grundlage mehrerer klinischer und experimenteller Studien wurde vorgeschlagen, dass ein Ungleichgewicht in der Expression von ERα und ERß als gemeinsames Merkmal einen kritischen Schritt bei der Östrogen-abhängigen Tumor-Progression darstellen könnte. Das Gleichgewicht zwischen dem ERα und dem ERß Rezeptor scheint für die Aufrechterhaltung einer normalen zellulären Funktion insofern von essentieller Bedeutung zu sein, als ERß als negativer Regulator der ERα-vermittelten Signalübermittlung wirkt, welche für die Proliferation verantwortlich ist. Dies wird auch durch Untersuchungen an ERβ-knock-out Mäusen belegt. Die Überexpression von ERα in diesen Mäusen resultiert in einem hyperproliferativen Uterus und führt zur Ovarialkarzinogenese (Frasor et al., Endocrinol. 2003; 144: 3159 - 3166).

Den ERα-vermittelten mitogenen Wirkungen von Östrogen schreibt man auch im Menschen eine kritische Rolle bei der Entwicklung und Progression von menschlichem Brust-, Endometrium- und Ovarialkrebs zu. Dies wurde kürzlich durch die Ergebnisse großer prospektiver Studien mit menopausalen Frauen gezeigt (Beral et al., Lancet 2002; 360: 942 - 944; Cheblowski et al., JAMA 2003; 289: 3243 - 3253; Writing Group for the Women's Health Initiative Investigators, JAMA 2002; 288: 321 - 333; Million Women Study Collaborators; Lancet 2003; 362: 419 - 427).

Es wird postuliert, dass die antiproliferative Wirkung von ERß durch Wechselwirkungen mit verschiedenen proliferativen Ereignissen vermittelt wird, was zu einer Schutzwirkung gegen eine ERα-induzierte Hyperproliferation führt. Insbesondere werden eine Inhibition der anti-apoptotischen Genexpression, eine Induktion der pro-apoptotischen Genexpression und eine Inhibition der ERα-induzierten Zellproliferation diskutiert (Bardin et al., Endocrine-Rel. Cancer 2004; 299: 408 - 414).

In verschiedenen Organen und Geweben, wie Brust, Ovarien, Uterus und Dickdarm, wurde beobachtet, dass unter den Bedingungen einer Abnahme der ERβ-Expression (z. B. bei Hyperplasie, neoplastischen Läsionen) eine unkontrollierte zelluläre Proliferation aufgrund einer ERα-Aktivierung erfolgt, was zu einem tumorigenen Status führt (Bardin et al., Endocrine-Rel. Cancer 2004; 11: 537 - 551). Somit könnte eine selektive Aktivierung von ERß bei der Prävention und/oder der Behandlung von Östrogen-abhängigen Tumoren, beispielsweise in der Brust, den Ovarien, des Uterus, der Prostata und des Colons, von therapeutischer Bedeutung sein.

Derzeitige Behandlungsstrategien für Brustkrebs im Frühstadium umfassen den selektiven Östrogenrezeptormodulator (SERM) Tamoxifen, der jedoch als ER-Antagonist nur bei ER-positivem Brustkrebs wirkt, bei ER-negativem Brustkrebs jedoch keine Wirkung zeigt (Park & Jordan, Trends Mol. Medicine 2002; 8: 82 - 88; Hanstein et al., Eur. J. Endocrinol. 2004; 150: 243 - 255). Darüber hinaus wurde beobachtet, dass die Langzeitbehandlung mit Tamoxifen aufgrund seiner agonistischen Aktivität am ERα Endometriumkrebs induziert (Stygar et al., Reprod. Biol. Endocrinol. 2003; 1 : 40). Daher darf Tamoxifen nicht länger als 2 Jahre zur postoperativen Nachsorge angewendet werden.

Östrogene üben zwar eine wichtige Funktion im Rahmen des normalen Wachstums, der Differenzierung und der Entwicklung der Prostata aus. Im Synergismus mit Androgenen sind Östrogene jedoch wirksame Induktoren eines aberranten Wachstums und einer neoplastischen Transformation der Prostata. Der ERα wird hauptsächlich in den Stroma-Kompartimenten der Prostata und in den Knochenmetastasen sowie den Metastasen der regionalen Lymphknoten exprimiert und man vermutet deshalb seine Beteiligung an der Pathogenese von Prostatakrebs.

Dagegen unterdrückt der ERß eine AR-vermittelte Hyperproliferation und fördert die Differenzierung der Prostataepithelzellen (Imamov et al., N.Engl. J. Med. 2004; 351: 2773-2774). Dies konnte auch an älteren ERβ-Knockout-Mäusen gezeigt werden, welche im Gegensatz zu den Wildtyp-Mäusen eine Prostatahyperplasie entwickelten (Krege et al., Proc. Natl. Acad. Sci 1998; 95: 15677-15682). In mehreren Studien konnte gezeigt werden, dass während der Prostatakarzinogenese der ERß herunterreguliert wird und dadurch eine ERβ-vermittelte Hemmung der Invasion und Apoptose der Tumorzellen nicht mehr im ausreichenden Maße aufrechterhalten werden kann (Weihua et al., Proc. Natl. Acad. Sci 2002; 99: 13589-13594; Cheng et al., FEBS Lett. 2004; 566: 169-172). Interessanterweise ist der endogene Ligand für den ERß in der Prostata nicht Östradiol, sondern 5α-Androstan-3β,17β-diol (3βAdiol oder 3β-Androstandiol), welches aus Dihydrotestosteron (DHT) gebildet wird und damit die Wirkung von DHT limitiert (Weihua et al., Proc. Natl. Acad. Sci 2002; 99: 13589-13594). Wird dieses Gleichgewicht zwischen AR- und ERβ-Aktivierung gestört, indem z.B. durch den 5-α-Reduktase-Inhibitor Finasterid die Bildung von DHT aus Testosteron, und damit auch die Produktion von 3βAdiol gehemmt wird, bleibt die ERβ-vermittelte Differenzierung des Prostataepithels aus. Dieser Mechanismus könnte für die höhere Rate an nicht differenzierten Tumoren in der Finasterid-Gruppe der Prostate Cancer Prevention Trial verantwortlich sein (Weihua et al. 2002). Zudem wurde kürzlich gezeigt, dass 5α-Androstan-3β,17β-diol durch die Aktivierung des ERß die Migration von Prostatakarzinomzellen hemmt und damit die Zellinvasion und Metastasierung verhindert (Guerini et al. Cancer Res 2005; 65:5445-5453). Daher erscheint es sinnvoll, zur Prostatakarzinomprävention oder -therapie Finasterid mit einem ERβ-Aktivator bei solchen Patienten zu kombinieren, bei denen der ERß im Prostatakarzinom noch detektierbar ist.

Die Proliferationsrate von Prostatakrebszellen ist ziemlich gering und folglich ist die Chemotherapie bei der Behandlung von Prostatakrebs von geringer Effizienz. Derzeitige Behandlungsstrategien umfassen Anti-Androgene, welche entweder Steroidderivate, wie Cyproteronacetat, oder nicht-steroidales Bicalutamid oder Flutamid darstellen. Diese Wirkstoffe sind jedoch nur bei hormon-sensitiven Prostatakrebs wirksam. Die häufigsten Nebenwirkungen dieser Wirkstoffe sind Gynäkomastie, kardiovaskuläre Erkrankungen, Müdigkeit, Appetitverlust, verringerte Libido und Spermatogenese sowie erhöhte Lebertoxizität.

Auch eine erhöhte Produktion von IL-6 ist ein weiterer wichtiger Faktor für die Entwicklung von Prostatakrebs (George et al. Clin Cancer Res 2005,11: 1815-1820). IL-6 aktiviert den Androgenrezeptor und führt damit zur Hyperproliferation des Prostataepithels. Auch in hormon-unabhängigen Prostatatumoren wird ein erhöhter IL-6-Spiegel als Prognosemarker angesehen. Daher wurde eine Therapie mit anti-IL-6-Antikörpern als aussichtsreich zur Reduktion der Morbidität angesehen. Jedoch befindet sich diese Therapie des Prostatakarzinoms noch im Entwicklungsstadium und ist zudem mit hohen Kosten verbunden.

Des Weiteren spielen sowohl IL-6 als auch endokrine Veränderungen eine wichtige Rolle bei der Entstehung der benignen Prostatahyperplasie bzw. des "lower urinary tract symptoms" (LUTS; Konig et al. Prostate. 2004, 58: 121-129; Donovan J. L., BJU Int. 2000, 85, Suppl. 1: 10 - 19). LUTS sind häufig mit einer benignen Prostataobstruktion (BPO) und sekundär mit einer benignen Prostatahyperplasie (BPH, Chapple, C.R., BJU Int. 2004; 94: 738 - 744) assoziiert. Dihydrotestosteron und Östradiol sind Schlüsselfaktoren dieser Erkrankung. Die 5-alpha-Reduktase ist das für die Umwandlung von Testosteron in 5-alpha-Dihydrotestosteron (DHT) verantwortliche Enzym. Somit ist prmär DHT, und nicht Testosteron für die Prostataentwicklung durch Aktivierung des AR verantwortlich. Die herkömmliche Behandlung von LUTS ist auf 5-alpha-Reduktase-Inhibitoren, z.B. Finasterid, und alpha₁-Blocker, z.B. Tamsulosin, fokussiert.

Typische Nebenwirkungen, wie verringerte Libido und Spermatogenese, bedingen jedoch eine geringe Compliance der Patienten zu Finasterid (Peters & Sorkin, Drugs 1993; 46: 177 - 208). Darüber hinaus reduziert Finasterid den Spiegel von Prostataspezifischem Antigen (PSA) und maskiert somit eine frühzeitige Diagnose eines Prostatakarzinoms (Peters & Sorkin, Drugs 1993; 46: 177 - 208). Unter den alpha₁Blockern ist Tamsulosin spezifisch auf den adrenergen alpha_{1A}-Rezeptor Subtyp gerichtet (Beduschi et al., Urology 1998; 51: 861 - 872; Dunn et al., Drugs Aging 2002; 19: 135 - 161). Obwohl Tamsulosin weniger Schwindelgefühl und andere kardiovaskuläre Nebenwirkungen als andere alpha₁-Blocker verursacht, besteht dennoch ein hohes Risiko für eine abnormale Ejakulation, wie z.B. retrograde Ejakulation, vermindertes Ejakulationsvolumen oder völliges Fehlen der Ejakulation (Lepor, Urol. 1998; 51 (6): 892 - 900).

Es besteht somit ein Bedarf an neuen Wirkstoffen für die Behandlung von endokrin-abhängigen und endokrin-unabhängigen Tumoren, wie z.B. Brust- und Prostatakrebs, oder Hyperplasien wie z.B. LUTS, wobei diese Wirkstoffe kein ERα-aktivierendes Potential in anderen Geweben und Organen zeigen sollten. Ein Weg bestünde in der Aktivierung von ERß als natürlichem Gegenspieler von ERα. Daher sind selektive Aktivatoren des ERß von Interesse, die nicht die beschriebenen Nebenwirkungen, insbesondere in der Langzeitanwendung, aufweisen.

Natürlich vorkommende Phytoöstrogene, wie die Sojaflavonoide Genistein und Daidzein, oder die Flavonoide Koumestrol zeigen eine gewisse Präferenz für ERβ. Sie aktivieren jedoch auch ERα *in vivo* (Kuiper et al., Endocrinol. 1997, 138: 863 - 870; Belcher & Zsarnovszky, J. Pharmacol. Exp. Therap. 2001; 299: 408 - 414), so dass eine Langzeitbehandlung, beispielsweise mit Soja-haltigen Präparaten in wirksamen Dosen, ein gewisses kanzerogenes Potential aufweisen könnte. Mit einem hochdosierten Soja-Präparat wurde nach einer 5-jährigen Anwendung bei Wechseljahresbeschwerden eine Endometriumhyperplasie beobachtet, die auf eine Aktivierung des ERα in diesem Gewebe zurückzuführen ist (Unfer. Fertil Steril 2004; 82: 145-148).

### 5. Weiterer Stand der Technik

Seit 1993 ist in Deutschland unter der Bezeichnung Extrakt Rheum rhaponticum (ERr 731^{®}) (Handelsname Phytoestrol^{®}N) ein Trockenextrakt aus Wurzeln von Rheum rhaponticum zur Follikelhormonersatztherapie, beispielsweise zur Behandlung von Frauen mit klimakterischen Beschwerden, juveniler Oligomenorrhoe und Dysmenorrhoe, primärer und sekundärer Amenorrhoe sowie Endometritis auf dem Markt. Die Inhaltsstoffe des spezifischen ERr 731^{®}-Extrakts sind Rhaponticin, Desoxyrhaponticin, Rhapontigenin und Desoxyrhapontigenin (Tabelle 1).

**Tabelle 1**

| Zusammensetzung des Extrakts ERr 731^{®} | | |
|---|---|---|
| **Hydroxystilben** | **Chemischer Name** | **CAS Nr.** |
| Rhaponticin | 3,3',5-Trihydroxy-4'-methoxystilben-3-O-ß-D-glucopyranosid | 155-58-8 |
| Desoxyrhaponticin | 3',5-Dihydroxy-4'-methoxystilben-3-O-β-D-glucopyranosid | 30197-14-9 |
| Rhapontigenin (Trans-Rhapontigenin) | 3,3',5-Trihydroxy-4'-methoxystilben | 500-65-2 |
| Desoxyrhapontigenin | 3',5-Dihydroxy-4'-methoxystilben | 33626-08-3 |

Sämtliche Inhaltsstoffe von ERr 731^{®} gehören zur Gruppe der Hydroxystilbene. Vertreter dieser Gruppe besitzen folgende allgemeine Formel:

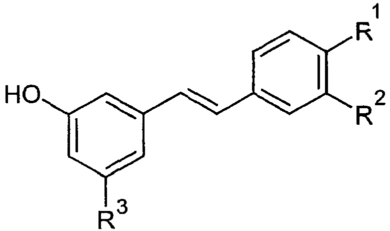

| | R¹ | R² | R³ |
|---|---|---|---|
| Resveratrol | OH | H | OH |
| Rhaponticin | OCH₃ | OH | O-Glc |
| Desoxyrhaponticin | OCH₃ | H | O-Glc |
| Rhapontigenin | OCH₃ | OH | OH |
| Desoxyrhapontigenin | OCH₃ | H | OH |
| Astringin | OH | OH | O-Glc |
| Piceatannol (Astringenin) | OH | OH | OH |

Mehrere Studien haben gezeigt, dass Zahl und Position der freien Hydroxy- und Methoxygruppen die biologische Aktivität der Hydroxystilbene stark beeinflusst (Kageura et al. Bioorganic & Medicinal Chemistry 9 (2001) 1887-1893, Matsuda et al. Biol. Pharm. Bull. 2001 (24(3) 264-267, Roberti et al. J. Med. Chem. 2003, 46, 3546-3554). Der pharmakologische Effekt der Hydroxystilbene ist darüber hinaus abhängig von der Präsenz einer Glukosegruppe (Park et al. Arch. Pharm. Res. 2002, 25(4), 528-533).

Ob, bzw. zu welchen Metaboliten die Inhaltsstoffe von ERr 731^{®}, z.B. nach oraler Verabreichung im Körper abgebaut werden, ist nur unzureichend untersucht. So ist lediglich aus Untersuchungen zur antithrombotischen und antiallergischen Aktivität von Rhaponticin-haltigen Extrakten aus Rhei Rhizoma bekannt, dass Rhaponticin durch Bakterien des menschlichen Intestinaltrakts zu Rhapontigenin abgebaut wird (Park et al, Arch. Pharm. Res. 2002, 25 (4), 528-533). Eine Metabolisierung von Rhaponticin zu Piceatannol oder von Desoxyrhaponticin zu Resveratrol wurde bisher nicht beschrieben.

Morris et al beschreiben in The Prostate 2002, 52, 319-329 die apoptotische Wirkung von reinem Resveratrol auf hormonsensitive und -nicht-sensitive Prostatakrebszellen und stellen die Vermutung an, dass eine Langzeitexposition durch die Aufnahme niedriger Resveratrol-Konzentration mit der Nahrung die Entwicklung von Prostatakrebs inhibieren könnte. Eine aktive präventive Behandlung durch Verabreichung eines Resveratrol-haltigen Mittels wird nicht vorgeschlagen.

Ashikawa et al beschreiben in J. Immunol. 2002, 6490-6497 die inhibierende Wirkung von Piceatannol auf den Transkriptionsfaktor NF-κB. Die Autoren sehen darin eine Erklärung für einen Antitumoreffekt von Piceatannol. Rhaponticin war dagegen bei diesen Tests inaktiv. Eine Verwendung von Piceatannol zur Behandlung von Prostatakrebs oder LUTS wird nicht vorgeschlagen.

Roberti et al beschreiben in J. Med. Chem. 2003, 46, 3546-3554 die apoptotische Wirkung von Resveratrol und Analogen auf HL60 Leukämiezellinien. Die wirksamsten Analoga waren dabei bestimmte cis-konfigurierte Derivate.

Gao et al beschreiben in The Prostate 2004 59, 214-225 die Modulation ARabhängiger Signalwege durch Resveratrol, welche mit Prostatakrebs in Zusammenhang gebracht werden. Je nach Konzentration wurde eine Steigerung oder eine Inhibierung der AR-abhängigen Genexpression beobachtet.

Aggarwal et al beschreiben in einem Übersichtsartikel (Anticancer Research, 24, 2004) die Rolle von Resveratrol bei der Prävention und Behandlung von verschiedenen Krebsformen, u.a. Prostatakrebs.

Die FR 2 835 185 beschreibt einen komplexen Rhabarberextrakt, erhältlich aus Wurzelstöcken von Rheum rhaponticum, der dadurch gekennzeichnet sein soll, dass er mindestens 50 % Hydroxystilbene enthält, wobei diese Hydroxystilbene zu mindestens 50 % aus Rhaponticin, Desoxyrhaponticin, Astrangin und Piceatannol bestehen. Ein bevorzugter Extrakt enthält 15 - 50 Gew.-% Rhaponticin, 10 - 35 Gew.-% Desoxyrhaponticin, 5 -10 Gew.-% Astrangin und 0,1 - 3 Gew.-% Piceatannol. Dieser Extrakt wird, wie in den Beispielen veranschaulicht, durch wässrig-alkoholische Extraktion von Wurzelstöcken von Rheum rhaponticum hergestellt. Der damit erzielbare Gesamtanteil an Rhaponticin und Desoxyrhaponticin beträgt dabei nur 76 Gew.-%. Astrangin ist in einem Anteil von 11 Gew.-%, Piceatannol in einem Anteil von 3 Gew.-% und Anthracenoside sind in einem Anteil von 0,5 Gew.-% enthalten. Darüber hinaus scheint dieser Extrakt ca. 10 Gew.-% weitere, nicht näher beschriebene Bestandteile zu enthalten. In der FR 2 835 185 wird außerdem behauptet, dass der dortige spezielle Extrakt infolge angeblicher Synergieeffekte der verschiedenen Inhaltsstoffe des Extraktes biologische Eigenschaften besitzt, die der Wirkung der einzelnen Hydroxystilbene erheblich überlegen sind, insbesondere denjenigen Wirkungen, die die dort beschriebenen Inhaltsstoffe einzeln aufweisen sollen. Der dort beschriebene Extrakt soll angeblich antioxidative, tumorhemmende, entzündungshemmende und östrogene Eigenschaften besitzen. Tatsächlich liefert aber die FR 2 835 185 keine nachprüfbare technische Lehre für die behauptete pharmakologische Anwendbarkeit, geschweige denn, für den behaupteten Synergieeffekt des dort beschriebenen komplexen Drogenextraktes. Der experimentelle Teil beschreibt lediglich einzelne Formulierungsbeispiele für Kapseln, Tabletten oder Cremes. Insbesondere fehlen jegliche Versuchsdaten, welche die angebliche Brauchbarkeit zur Behandlung von solchen Erkrankungen belegen, welche im Zusammenhang mit freien Radikalen stehen, wie zum Beispiel beschleunigte Alterung, Krebs, Arteriosklerose, Falten, entzündliche Phänomene und dergleichen. Auch die behauptete Eignung einer Kombination des dort beschriebenen Rhabarberextrakts mit einem an Prenylflavonoiden reichen Hopfenextrakt zur Behandlung von mit freien Radikalen stehenden Krankheiten und/oder zur Behandlung von hormonellem Ungleichgewicht, wie Amenorrhoe, Menopause, Hitzewallungen usw. ist durch keine Daten belegt. Es bleibt darüber hinaus völlig unklar, welche der tatsächlich in dem dort beschriebenen Extrakt enthaltenen Komponenten (Rhaponticin, Desoxyrhaponticin, Astrangin, Piceatannol, Anthracenoside sowie die in einem Anteil von 10 % enthaltenen nicht analysierten Bestandteile) zu der behaupteten pharmakologischen Aktivität beitragen oder gegebenenfalls sogar für die behauptete Synergie zwingend erforderlich sind. Die tatsächliche Offenbarung der FR 2 835 185 sollte daher auf die Herstellung eines spezifischen, komplexen Rhabarberextraktes durch wässrig-alkoholische Extraktion von Wurzelstöcken von Rheum rhaponticum und die Herstellung spezifischer Hydroxystilben-Derivate sowie die Herstellung diverser pharmazeutischer Formulierungen beschränkt sein.

### ZUSAMMENFASSUNG DER ERFINDUNG

Der vorliegenden Efindung lag daher die Aufgabe zugrunde einen neuen Weg zur Prävention und/oder Behandlung von Prostatakrebs und/oder von Lower Urinary Tract Symptoms (LUTS) zu finden.

Diese Aufgabe wurde überraschenderweise durch die Verwendung einer Hydroxystilben-haltigen Wirkstoffkombination gemäß der Definition in den beiliegenden Patentansprüchen zur Herstellung eines pharmazeutischen Mittels gelöst, die zur Behandlung der oben genannten Gruppe von Erkrankungen überraschend vorteilhaft geeignet ist.

Die vorliegende Erfindung beruht auch auf der Erkenntnis einer neuen Wirkungsweise erfindungsgemäßer Wirkstoffe und Wirkstoffkombinationen, wie insbesondere des Trockenextraktes ERr 731^{®} sowie seiner Inhaltsstoffe und Metabolite.

### FIGURENBESCHREIBUNG

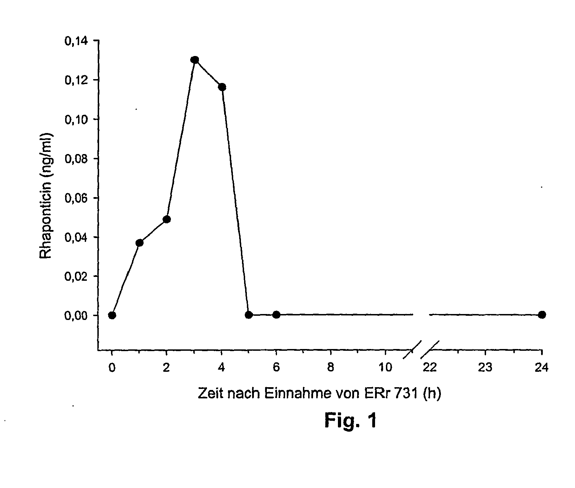

Figur 1 zeigt das Ergebnis einer pharmakokinetischen Untersuchung des Inhaltsstoffs Rhaponticin von ERr 731^{®} im Blut einer Probandin nach oraler ERr 731^{®} Gabe. Rhaponticin konnte im Blut nachgewiesen werden, nicht jedoch Rhapontigenin. Ebenso war dessen Metabolit Piceatannol unter den Versuchsbedingungen im Blut nicht nachweisbar.

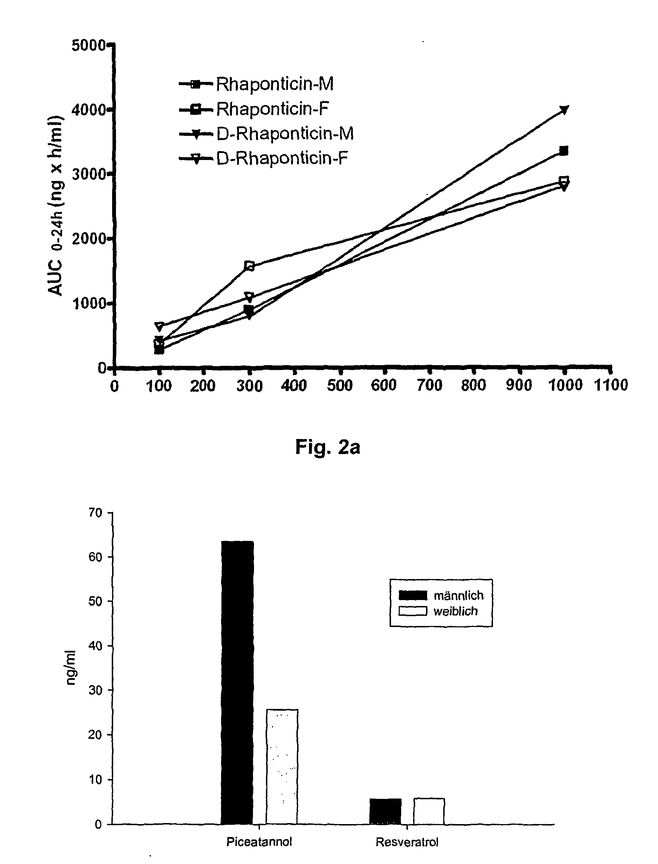

Figur 2a zeigt die dosisabhängige Akkumulation (AUC 0-24h (ng x h/ml)) von Rhaponticin und Desoxyrhaponticin im Hundeplasma (M = male, F = female) nach Verabreichung von ERr 731^{®}, die Aglycone Rhapontigenin und Desoxyrhapontigenin sind nicht detektierbar; Figur 2b zeigt die Bildung von Piceatannol und Resveratrol in vivo in männlichen und weiblichen Hunden 24 Stunden nach Verabreichung von 100 mg ERr 731^{®}/kg Körpergewicht.

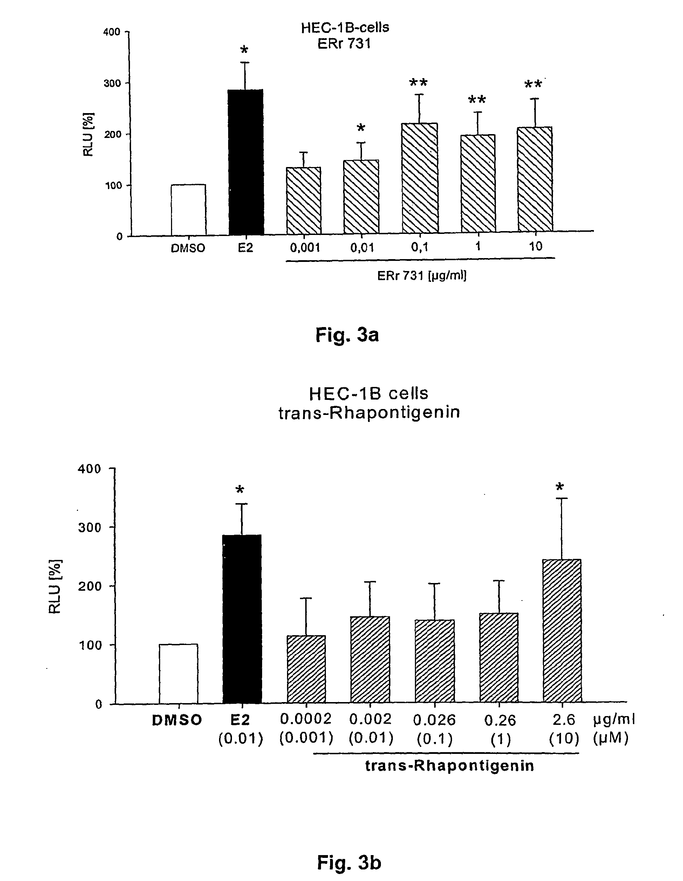

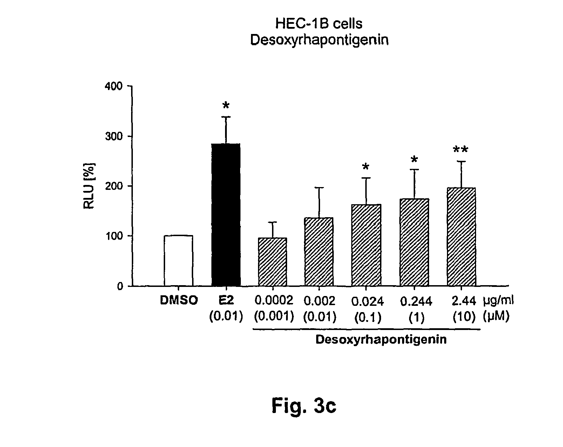

Figur 3 zeigt das Ergebnis von Versuchen zur Aktivierung des Östrogenrezeptors-β (ERβ) durch die Wirkstoffkombination ERr 731^{®} in der humanen Endometriumkarzinomzelllinie HEC-1B (Figur 3a); die Aglycone trans-Rhapontigenin (Figur 3b) und Desoxyrhapontigenin (Figur 3c) sind erst bei höheren Konzentrationen wirksam (E2 = Estradiol; RLU = relative Luciferase Units); * = p < 0.05; ** = p < 0.01

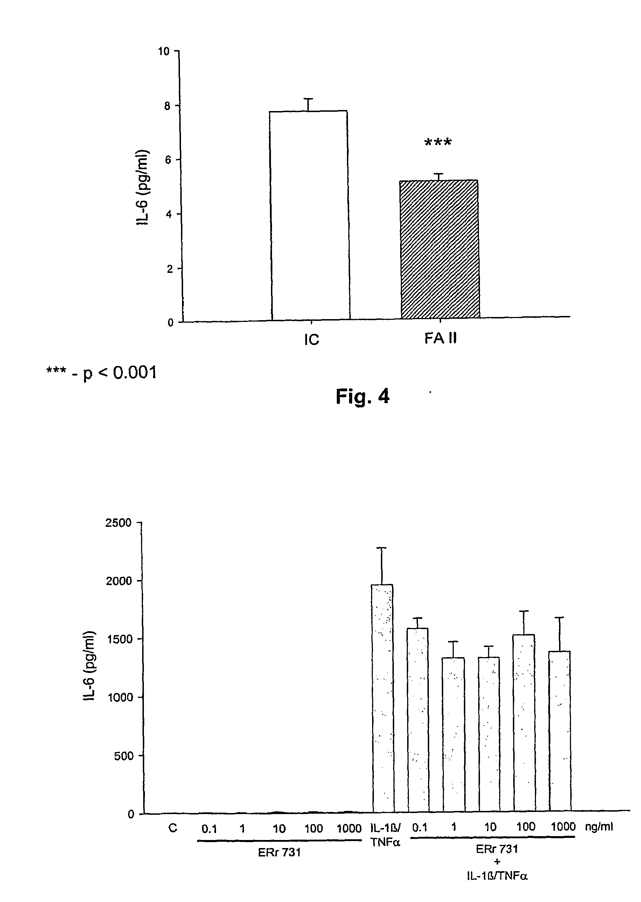

Figur 4 zeigt die Wirkung einer 15-monatigen Behandlung von Patientinnen mit klimakterischen Beschwerden (FA II) mit von ERr 731^{®} auf den IL-6 Spiegel. Es wurde festgestellt, dass mit ERr 731^{®} die IL-6-Spiegel gegenüber den Spiegeln vor der ersten Einnahme (IC) signifikant reduziert waren; ***= p < 0.001.
Figur 5 zeigt die Reduktion der durch Gabe der Cytokine IL-1β und TNFα stimulierten IL-6 Produktion in der humanen Lungenkarzinomzelllinie A549 durch die Wirkstoffkombination ERr 731^{®}.

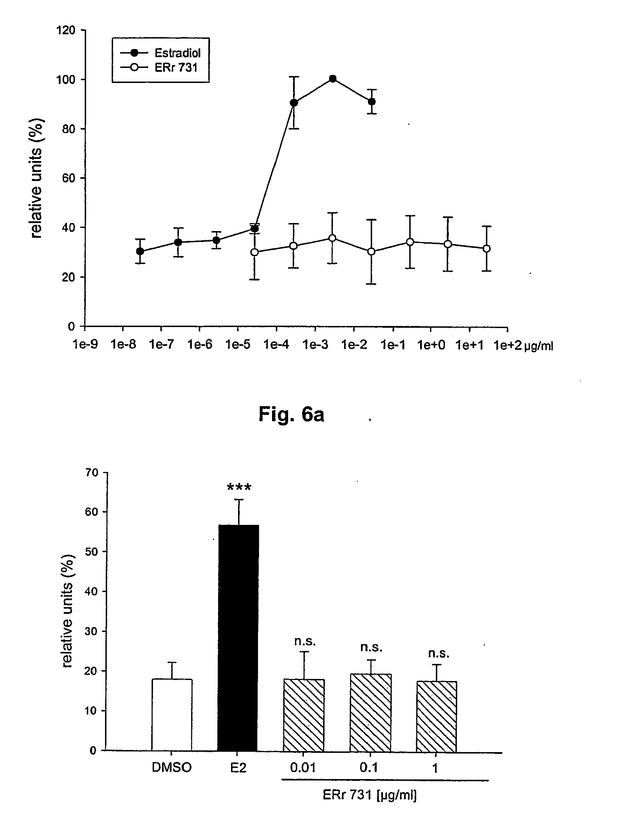

Figur 6 zeigt des Versuchsergebnis zur ERα-Aktivierungs mit ERr 731^{®}. Die Wirkstoffkombination aktiviert den ERα weder in der humanen Endometriumkarzinomlinie Ishikawa (Figur 6b) (***= p < 0.001) noch in Hefezellen (Figur 6a), die beide mit dem ERα transfiziert wurden.

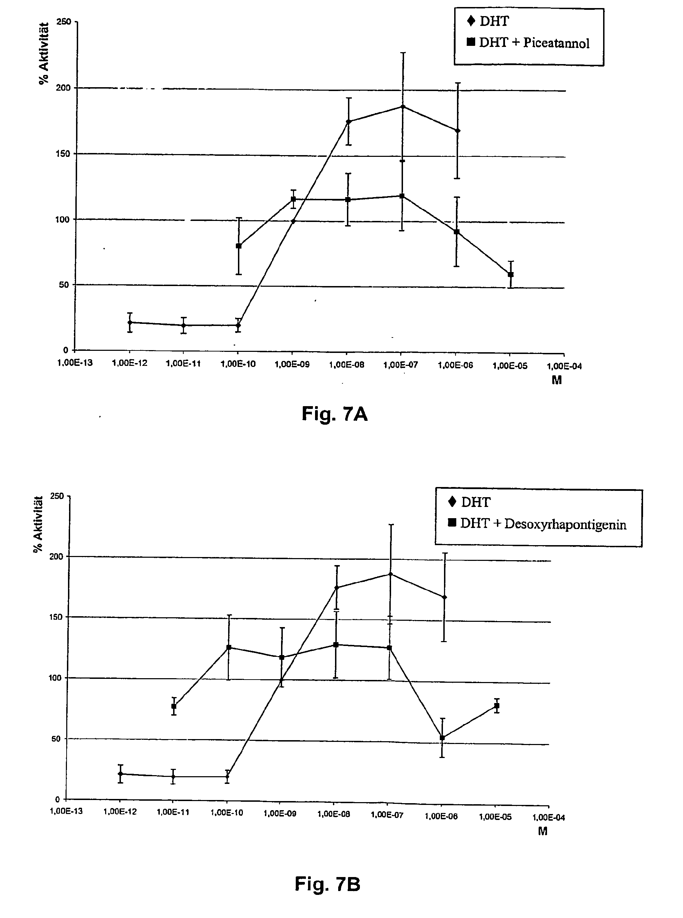

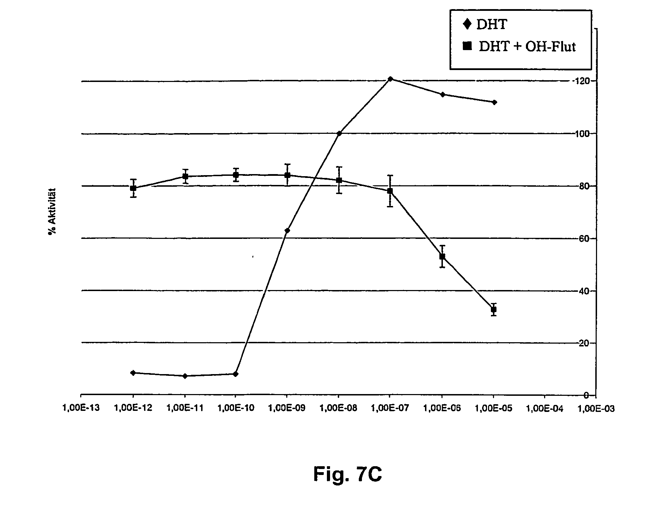

Figur 7 zeigt die antiandrogene Aktivität verschiedener Hydroxystilbene in einem Androgenrezeptor-exprimierenden Hefezellsystem; Fig. 7A: Dihydrotestosteron (DHT) in Anwesenheit von Piceatannol; Fig. 7B: DHT in Anwesenheit von Desoxyrhapontigenin Fig. 7C: DHT in Anwesenheit von Hydroxyflutamid (positive Kontrolle).

### DETAILLIERTE BESCHREIBUNG DER ERFINDUNG

### 1. Bevorzugte Gegenstände der Erfindung

Ein erster Gegenstand der Erfindung betrifft die Verwendung einer Hydroxystilben-haltigen Wirkstoffkombination wobei die Wirkstoffkombination im Wesentlichen Rhaponticin und Desoxyrhaponticin umfasst, zur Herstellung eines Mittels zur Behandlung von Prostatakrebs und/oder von Lower Urinary Tract Symptoms (LUTS), wobei Rhaponticin und Desoxyrhaponticin in einem Gewichtsverhältnis von etwa 10:1 bis 1:10 enthalten sind.

Gegenstand der Erfindung ist auch die Verwendung einer Hydroxystilben-haltigen Wirkstoffkombination nach Anspruch 1 umfassend zusätzlich gegebenenfalls Rhapontigenin, Desoxyrhapontigenin und Astringin; sowie deren stereoisomeren Formen; jeweils in Form ihrer Salze oder in der Phenolform, oder funktionellen Derivaten davon.

Insbesondere ist die erfindungsgemäß Verwendung findende Wirkstoffkombination gekennzeichnet durch
(1) einen Hydroxystilben-Gesamtgehalt von mehr als 90 Gew.-%; und/oder
(2) einem Aglycon-Anteil von weniger als 5 Gew.-%; und/oder
(3) einen Gehalt von weniger als 0,5 Gew.-% an Anthrachinon und/oder Anthrachinoiden ,
wobei die Prozentangaben jeweils auf Trockengewicht der Wirkstoffkombination bezogen sind

Erfindungsgemäße Mittel sind dabei insbesondere ausgewählt unter Arzneimitteln, wie z.B. Homöopathika, andere Arzneipflanzenzubereitungen, Nahrungsergänzungsmitteln, diätetischen Lebensmitteln.

Vorzugsweise liegen die Wirkstoffe im Wesentlichen in der trans-Form vor. Salze sind insbesondere die Alkali- und Erdalkaliphenolate obiger Verbindungen, die eine oder mehrere freie phenolische Hydroxylgruppen aufweisen. Liegen mehrere Hydroxylgruppen vor, so können diese teilweise oder vollständig in der Salzform vorliegen.

Der erfindungsgemäß einzusetzende Wirkstoff oder die Wirkstoffkombination ist dabei chemisch synthetisiert oder insbesondere aus natürlichen oder rekombinanten Pflanzen isolierbar. Funktionelle Derivate im Rahmen der Erfindung sind insbesondere solche Derivate, die im menschlichen oder tierischen Körper nach Verabreichung wieder in die nichtderivatisierte Ausgangsverbindung zurückführbar sind. Insbesondere sind zu nennen Ether und Esterderivate. Dabei können einzelne oder alle veretherbaren oder veresterbaren Gruppen eines Moleküls (insbesondere die phenolischen und glucosidischen Hydroxygruppen) derivatisiert sein. Beispiele geeigneter Derivate und deren Herstellung sind z.B. in der FR 2 835 185 beschrieben, worauf hiermit ausdrücklich Bezug genommen wird. So können genannt werden: Ester von gesättigten oder ungesättigten, aliphatischen oder aromatischen Carbonsäuren mit bis zu 25 Kohlenstoffatomen, wie 1 bis 25 Kohlenstoffatomen, wie z.B. gesättigte C₆ - C₂₂-Fettsäuren (wie z.B. gesättigte unverzweigte Fettsäuren ausgewählt unter Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure); oder Silylether, wobei das Siliziumatom drei gleiche oder verschiedene, geradkettige oder verzweigte, gesättigte oder ungesättigte Kohlenwasserstoffreste mit bis zu 20 Kohlenstoffatomen, wie z.B. C₁ - C₂₀-Alkyl oder C₂ - C₂₀-Alkenyl, trägt.

In einer bevorzugten Ausführungsform ist der Wirkstoff oder die Wirkstoffkombination aus Pflanzen zugänglich, die ausgewählt sind unter natürlichen Pflanzen sowie durch Züchtung veränderten oder rekombinanten, genetisch veränderten, Pflanzen, die einen in Vergleich zur entsprechenden unveränderten Pflanze höheren Gehalt an wenigstens einem der gewünschten Inhaltsstoffe aufweisen. Insbesondere sind diese Pflanzen ausgewählt unter Pflanze der Gattung Rheum spp., Astragalus spp., Cassia spp. oder Picea spp. oder wirkstoffhaltigen Pflanzenteilen. Nichtlimitierende Beispiele für geeignete Arten dieser Gattungen sind Rheum undulatum, Rheum palmatum, Rheum tataricum, Rheum officinale, Rheum wittrockii, Rheum altaicum, Rheum reticulatum, Astragalus complanatus, Cassia garrettiana und Picea sitchensis.

Dem Fachmann ist außerdem bewusst, dass Gattungen/Arten unterschiedlicher Provinienz sowie verschiedenen Alters (d.h. Ernte zu verschiedenen Zeiten der Vegetationsperiode) einsetzbar sind, was wiederum Einfluss auf Art, Menge und Zusammensetzung des daraus isolierbaren Wirkstoffe und Gemische haben kann. Ebenso sind verschiedene Pflanzenteile, wie Wurzeln, Rhizome, Blätter und/oder Stängel, grundsätzlich brauchbar.

Besonders vorteilhaft ist der Wirkstoff oder die Wirkstoffkombination erhältlich aus den Wurzeln, insbesondere von Rheum rhaponticum.

Die Wirkstoffkombination umfasst im Wesentlichen Rhaponticin und Desoxyrhaponticin in einem Gewichtsverhältnis von etwa 10:1 bis 1:10, wie z.B. in einem Bereich von etwa 5:1 bis 1:5 oder 4: 1 bis 1:4 oder 3:1 bis 1:3 oder 2:1 bis 1:2 oder etwa 1:1.

Eine weitere bevorzugte Wirkstoffkombination kann Rhaponticin und Desoxyrhaponticin, insbesondere in oben angegebenen Mengenverhältnissen, sowie Rhapontigenin und/oder Desoxyrhapontigenin umfassen. Der mengenmäßige Anteil von Rhapontigenin und/oder Desoxyrhapontigenin am Gesamtwirkstoffgehalt kann über einen weiten Bereich schwanken, und liegt z.B. im Bereich von etwa 0.01 bis 20 Gew.-%, insbesondere 0.1 bis 5 Gew.-%, bezogen auf den Gesamtwirkstoffgehalt.

Weiterhin sind Wirkstoffkombinationen bevorzugt, die einen Hydroxystilben-Gesamtgehalt, insbesondere einen Gesamtgehalt an Desoxyrhaponticin, Desoxyrhapontigenin, Rhaponticin und Rhapontigenin, oder einen Gesamtgehalt an Rhaponticin und Desoxyrhaponticin, von mehr als 90 Gew.-%, wie z.B. 91 bis 100 Gew.-%, oder 92 bis 99 oder 93 bis 98 oder 94 bis 97 Gew.-%, aufweisen.

In einer weiteren bevorzugten Ausführungsform wird eine Wirkstoffkombination verwendet, die im wesentlichen frei ist von Aglycon-Derivaten von Rhaponticin und Desoxyrhaponticin, wie insbesondere Resveratrol und Piceatannol. "Im Wesentlichen frei" bedeutet einen Aglycon-Gehalt von weniger als 5 Gew.-%, insbesondere weniger als 2 Gew.-% wie z.B. weniger als 1 Gew.-% oder 0,1 Gew.-%, wie 0 bis 0,05 Gew.-%, jeweils bezogen auf den Gesamtgehalt an Rhaponticin und Desoxyrhaponticin.

In einer weiteren bevorzugten Ausführungsform wird als Wirkstoffkombination ein pflanzlicher Trockenextrakt verwendet, der einen hohen Anteil an Glycosiden, insbesondere Glycosiden des oben beschriebenen Typs, aufweist. Glycoside sind insbesondere glycosidische Vorläufer von Resveratrol und Piceatannol. Resveratrol- und Piceatannol-"Prodrugs" im Sinne der Erfindung sind insbesondere Substanzen, welche in vivo, wie z.B. im Menschen und/oder einem anderen Säuger, wie z.B. Hund, in Resveratrol und/oder Piceatannol teilweise oder vollständig überführbar sind. Hierbei kann es sich um zuckerhaltige (Glycone, Glycoside) oder zuckerfreie (Aglycone) natürliche oder synthetische "vorläufer" von Resveratrol oder Piceatannol handeln. Typische Beispiele für zuckerhaltige Vorläufer umfassen Rhaponticin, Astringin und Desoxyrhaponticin. Typische Beispiele für zuckerfreie Vorläufer umfassen Rhapontigenin und Desoxyrhapontigenin. Die Begriffe "Prodrug" oder Vorläufer" sind aber im Kontext der Erfindung nicht als funktionelle Einschränkung zu verstehen. Wie durch die später beschriebenen Versuchsergebnisse belegt, entfalten insbesondere die erfindungsgemäßen "Vorläufer" per se vorteilhafte pharmakologische Wirkungen. Diese sind z.B. in einem Anteil von 30 bis 100 Gew.-%, 50 bis 100 Gew.-%, vorzugsweise jedoch in Anteilen vor mehr als 76 Gew.-%, wie 76 bis 99 Gew.-% oder 80 bis 98 Gew.-% oder 85 bis 96 Gew.-%, jeweils bezogen auf das Gesamtgewicht des Trockenextrakts, enthalten.

Weiterhin sind solche Wirkstoffkombinationen bevorzugt, die einen Gehalt von weniger als 0,5 Gew.-% , wie z.B. 0 - 0,49 Gew.% oder 0,001 bis 0,3 oder 0,01 bis 0,2 oder 0,01 bis 0,1 Gew.-% an Anthrachinon und/oder Anthrachinoiden (jeweils bezogen auf Trockengewicht der Wirkstoffkombination) aufweisen. Anthrachinoide sind dabei im weitestens Sinn als Substanzen mit Anthrachinon-Grundgerüst zu verstehen.

Ein Trockenextrakt" in Sinne der Erfindung liegt insbesondere dann vor, wenn der Restfeuchtegehalt, d.h. der Restanteil an Wasser und/oder organischer Flüssigkeit, (wie Extraktionsmittel) weniger als etwa 5 Gew.-%, insbesondere weniger als 2 Gew.-%, wie z.B. 0 bis 1,5 Gew.-% oder 0,1 bis 0,5 Gew.-%, jeweils bezogen auf das Gesamtgewicht des erhaltenen Trockenextrakts, beträgt.

Nichtlimitierende Beispiele für eine geeignete Wirkstoffkombination, umfassend die Wirkstoffe Rhaponticin, Desoxyrhaponticin, Rhapontigenin, Desoxyrhapontigenin, werden im folgenden aufgeführt
60-70 Gew.-%, wie z.B. 60-66 oder 62-68 Gew.-%, Rhaponticin
30-40 Gew.-%, wie z.B. 30-36 oder 31-37 Gew.-%, Desoxyrhaponticin
0-2 Gew.-% trans-Rhapontigenin und
0-2 Gew.-% Desoxyrhapontigenin;
oder
50-60 Gew.-%, wie z.B. 53-58 Gew.-%, Rhaponticin
20-30 Gew.-%, wie z.B. 14-28 Gew.-%, Desoxyrhaponticin
5-20 Gew.-%, wie z.B. 10-18 Gew.-%, trans-Rhapontigenin und
0-10 Gew.-%, wie z.B. 4-10 Gew.-%, Desoxyrhapontigenin;
jeweils bezogen auf den Gesamtwirkstoffgehalt und insbesondere auf den Gesamtgehalt an Rhaponticin, Desoxyrhaponticin, Rhapontigenin und Desoxyrhapontigenin.

Die oben beschriebenen Wirkstoffkombinationen eignen sich insbesondere zu Verwendung zu folgenden medizinischen Zwecken:
a) Prävention und/oder Behandlung von Prostatakrebs und LUTS, wie benigner Prostataobstruktion und/oder benigner Prostatahyperplasie.
b) Prävention und/oder Behandlung von leichter bis mittelschwerer Depressionen und Angstzuständen; insbesondere Behandlung leichter bis mittelschwerer Depressionen und Angstzustände bei weiblichen menopausalen oder nichtmenopausalen Patientinnen oder männlichen Patienten;

Die Bestimmung des Schweregrads von Depressionen und Ängstlichkeit kann dabei nach in der Fachwelt anerkannten Standards, wie dem HAMA - Anxiety-Scale, Beck Anxiety Inventory -Skala (BAI), Beck Depression Inventory-Skala (BDI-II) oder dem HAMD - Depression-Scale, erfolgen.
c) Prävention und/oder Behandlung von chronisch-entzündlicher Erkrankungen, wobei die entzündliche Erkrankung insbesondere ausgewählt ist unter Osteoarthritis, rheumatoider Arthritis, juveniler idiopathischer Arthritis, Psoriasis-Arthritis, Psoriasis vulgaris; Endometriose, Blasen-, Harn- und Nierenentzündungen sowie inflammatorischen Darmerkrankungen, wie Morbus Crohn und Colitis ulcerosa.
d) Prävention und/oder Behandlung von endokrin-abhängigen Tumoren, ausgewählt unter Tumoren der Brust, der Ovarien, des Uterus, der Prostata und des Colons, wobei der endokrin-unabhängige Tumor ausgewählt ist unter Tumoren der Brust, der Ovarien, des Uterus, der Prostata und des Colons
e) Prävention und/oder Behandlung von Migräne,
wobei die Verwendung zu den medizinischen Zwecken b), c), d) und e) nicht Gegenstand der vorliegenden Erfindung ist.

Gegenstand der Erfindung ist auch die Verwendung von Kombinationen von Wirkstoffen nach obiger Definition, in Kombination mit wenigstens einem weiteren zur Prävention und/oder Behandlung einer der oben unter a) genannten Erkrankungen geeigneten und von Verbindungen gemäß obiger Definition verschiedenen Wirkstoff. Insbesondere ist auch eine Kombination mit Vitaminen, Mineralstoffen, weiteren Arzneipflanzenzubereitungen und/oder Nahrungsergänzungsmitteln und/oder diätetischen Lebensmitteln möglich.

Gegenstand der Erfindung ist auch eine Dosierungsform, enthaltend in einem pharmazeutisch verträglichen Träger eine Wirkstoffkombination gemäß den beiliegenden Ansprüchen zur Verwendung nach den beiliegenden Ansprüchen.

Geeignete feste Dosierungsformen weisen einen Gesamt-Wirkstoffgehalt von etwa 1 bis 20 mg, wie z.B. 2 bis 10 mg, je Dosiseinheit auf.

Insbesondere weisen geeignete Dosierungsformen einen zuckerfreien, insbesondere, Mono- oder Disaccharid-freien, wie z.B. Lactose-freien Kern auf.

Geeignete feste Dosierungsformen können in Form einer Pille, einer Tablette, eines Extrudats oder eines Granulats vorliegen.

Feste Dosierungsformen in Form eines Dragees, gegebenenfalls mit einem magensaftresistenten Überzug sind ebenfalls geeignet. Vorzugsweise sind derartige Überzüge frei von Weichmachern, wie Phthalaten, wie z.B. Diethylphthalat. Beschichtungsmittel, welche sich besonders zur Herstellung magensaftresistenter, weichmacherfreier Überzüge eignen, sind ausgewählt unter bekannten natürlichen und synthetischen Beschichtungsmitteln (vgl. z.B. Voigt, Pharmazeutische Technologie, 7. Auflage 1993, Ullstein Mosby, Berlin). Besonders geeignete Beschichtungsmittel sind, ohne darauf beschränkt zu sein, Schellack und Cellulosederivate, wie Hydroxypropylmethylcellulosederivate, wie z.B. Hydroxypropylmethylcelluloseacetatsuccinat, erhältlich unter der Handelsbezeichnung AQOAT.

Insbesondere ist eine feste Dosierungsform mit einem Gesamtgewicht im Bereich von etwa 150 mg ± 20 mg einem Kerngewicht von 84 mg ± 10 mg und einem Wirkstoffgehalt von etwa 3 bis 10 mg zu nennen.

Geeignet sind weiterhin feste Dosierungsformen, wobei diese eine Gleichförmigkeit des Wirkstoffgehalts (gemittelt über 10 oder 20 zufällig ausgewählte einzelne Dosiseinheiten) maximal ± 5 Gew.-%, wie z.B. ± 0,1 bis 4 oder ± 0,5 bis 3 oder ± 1 bis 2 Gew.-%, bezogen auf das Gesamtgewicht der Dosiseinheit aufweisen (z.B. bestimmt nach Ph. Eur. 5. Ausgabe 2005 (5.0/2.09.06.00)).

Eine geeignete feste Dosierungsform kann mittels eines Verfahrens hergestellt werden, wobei man
a) die Wirkstoffkombination mit dem pharmazeutisch verträglichen Träger mischt; und
b) die Mischung zum Wirkstoffkern verfestigt.

Vorzugsweise wird dazu die Wirkstoffkombination in einer inerten Flüssigkeit gelöst oder dispergiert und mit dem Träger gemischt und das Lösungsmittel während oder nach der Verfestigung entfernt.

VorteiJhafterweise stellt man die Wirkstoffkombination dadurch her, dass man
a) einen Wirkstoff-haltigen Teil einer Drogenpflanze, gegebenenfalls in zerkleinerter Form, bereitstellt,
b) diesen mit einem wässrigen Extraktionsmittel versetzt,
c) nach Einwirken des Extraktionsmittels eine Flüssigextraktphase aus der Mischung gewinnt und die Extraktion gegebenenfalls mehrfach wiederholt, und
d) das Extraktionsmittel aus den so erhaltenen Flüssigextraktphasen entfernt.

Vorzugsweise führt man dabei eine Extraktion mit einem wässrigen Extraktionsmittel bei einem pH-Wert der Mischung im alkalischen Bereich durch.

Die extrahierte Drogenpflanze ist insbesondere unter Pflanzen der Gattung Rheum spp, Astragalus spp,Cassia spp oder Picea spp ausgewählt.

In einer bevorzugten Variante des Herstellungsverfahrens mischt man die Gesamtmenge der Wirkstoffkombination portionsweise mit dem pharmazeutisch verträglichen Träger, wie z.B. Avicel oder einem vergleichbaren Träger auf Cellulosebasis, insbesondere mikrokristalliner Cellulose, und wiederholt den Mischvorgang nach jeder Trägerzugabe, mindestens jedoch ein oder zweimal. Insbesondere wird dabei mit einer Kugelmühle über eine Dauer von 30 Minuten bis 3 Stunden, wie z.B. 1 bis 2 Stunden gemischt. Beispielsweise kann man zum Mischen übliche Laborkugelmühlen, wie in den Beispielen beschrieben, verwenden. Dadurch erhält man eine homogene und stabile Verteilung des Wirkstoffs im Träger.

Nach einer weiteren Verfahrensvariante versieht man den Wirkstoffkern mit einem magensaftresistenten, vorzugsweise weichmacherfreien, Überzug.

Nach einer weiteren bevorzugten Variante wird dabei der Kern dragiert.

Geeignet sind auch flüssige Dosierungsformen, enthaltend eine Wirkstoffkombination gemäß obiger Definition einem Anteil von etwa 0,1 bis 20 mg/ml, wie z.B. 0,5 bis 15 oder 1 bis 10 oder 2 bis 5 mg/ml, in einem Lösungsmittelgemisch, umfassend Wasser und einen pharmazeutisch verträglichen Alkohol, wie insbesondere Ethanol. Vorzugsweise ist das Lösungsmittelgemisch ein Wasser/Ethanolgemisch mit einem Ethanolanteil von 10 bis 50 oder 20 bis 40 oder 25 bis 35 Vol.-%, wie z.B. 30 Vol.-%. Diese flüssigen Dosierungsformen sind insbesondere als Tropfen zur oralen Verabreichung formuliert.

Geeignet sind auch halbfeste Dosierungsformen, enthaltend eine Wirkstoffkombination gemäß obiger Definition in einem Anteil von etwa 1 bis 12, insbesondere 2 bis 6 mg Wirkstoffkombination (pro Gramm der Formulierung) in einem üblichen halbfesten Träger. Geeignet gelbildende Träger sind allgemein bekannt und z.B. ausgewählt unter quellbaren Cellulosederivaten, wie Hydroxypropylmethylcellulose, oder Polyacrylaten, wie z.B. Carbopol, oder Gelatine.

Geeignet sind auch Mittel, umfassend eine feste, halbfeste oder flüssige Dosierungsformen nach obiger Definition. Mittel im Sinne der Erfindung sind insbesondere pharmazeutische Mittel oder Arzneimittel, wie z.B. Homöopathika, sowie Arzneipflanzenzubereitungen.

Ein weiterer Gegenstand der Erfindung betrifft die Verwendung einer festen, halbfesten oder flüssigen Dosierungsform nach obiger Definition oder hergestellt nach einem der oben beschriebenen Verfahren zur Herstellung eines Mittels gemäß den beiliegenden Ansprüchen zur Behandlung von Prostatakrebs und/oder von LUTS. Aufgrund der ausgezeichneten Verträglichkeit der oben beschriebenen Wirkstoffe bzw. Wirkstoffkombinationen ist Gegenstand der Erfindung auch die Verwendung im Rahmen einer Langzeittherapie, die zeitlich unbegrenzt erfolgen kann. Die dabei zu verabreichende tägliche Dosis kann im Bereich von 0,1 bis 20 mg oder 0,5 bis 15 mg, 1 bis 10 oder 4 bis 8 mg Wirkstoffkombination, wie z.B. ERr 731^{®} liegen.

### 2. Weitere spezielle Ausgestaltungen erfindungsgemäß verwendeter Formulierungen

### 2.1 Arzneimittel

Die Erfindung umfasst auch die Herstellung pharmazeutischer Mittel (Arzneimittel) zur Verwendung zum oben genannten medizinischen Zweck a) zur Behandlung eines Individuums, vorzugsweise eines Säugers, insbesondere eines Menschen, Nutz- oder Haustieres. So werden die oben beschriebenen Wirkstoffkombinationen gewöhnlich in Form von pharmazeutischen Zusammensetzungen verabreicht, die einen pharmazeutisch verträglichen Exzipienten mit einem Gemisch mehrerer erfindungsgemäßer Wirkstoffe, und gegebenenfalls weitere Wirkstoffe umfassen. Diese Zusammensetzungen können beispielsweise auf oralem, lokalem, rektalem, transdermalem, subkutanem, intravenösem, intramuskulärem, intraperitonealem, intrakutanem oder intranasalem Weg verabreicht werden.

Beispiele geeigneter pharmazeutischer Formulierungen sind feste Arzneiformen, wie Pulver, Puder, Granulate, Tabletten, wie überzogene Tabletten, magensaftresistente überzogene Tabletten, Mantel-, Punkt- und Schichttabletten, Pastillen, Kautabletten, Lutschtabletten, Sachets, Cachets, Dragees, Kapseln wie Hart- und Weichgelatinekapseln, Globuli, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere ÖI-in-Wasser-Emulsionen, Suspensionen, beispielsweise Lotionen, Injektions- und Infusionszubereitungen, Augen- und Ohrentropfen, Nasentropfen, Nasenspray und Tinkturen. Auch implantierte Abgabevorrichtungen können zur Verabreichung erfindungsgemäßer Inhibitoren verwendet werden. Ferner können auch Liposomen, Mikrosphären oder Polymermatrizes zur Anwendung kommen.

Bei der Herstellung der Zusammensetzungen werden erfindungsgemäße Wirkstoffkombinationen gewöhnlich mit einem Exzipienten vermischt oder verdünnt. Exzipienten können feste, halbfeste oder flüssige Materialien sein, die als Vehikel, Träger, Adsorbens oder Medium für den Wirkstoff oder die Wirkstoffkombinationen dienen.

Zu geeigneten Exzipienten gehören beispielsweise Lactose, Dextrose, Succrose, Sorbitol, Mannitol, Stärken, Akaziengummi, Calciumphosphat, Alginate, Traganth, Gelantine, Calciumsilikat, mikrokristalline Cellulose, Polyvinylpyrrolidon, Cellulose, Cellulosederivate, wie z.B. Methylcellulose, Wasser, Sirupe und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel; Emulsionsstabilisatoren Filmbildner; Gelbildner; Geruchsmaskierungsmittel; Geschmackskorrigentien; Harze; Hydrokolloide; Lösemittel; Lösungsvermittler, Neutralisierungsmittel; Permeationsbeschleuniger; Pigmente; quaternäre Ammoniumverbindungen; Rückfettungs- und Überfettungsmittel; Salben-, Creme- oder ÖI-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen.

Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist; vgl auch Hager's Handbuch der Pharmazeutischen Praxis, Springer Verlag, Heidelberg.

Erfindungsgemäß geeignete Lösungsmittel für die Herstellung von Formulierungen sind insbesondere zu nennen ein- oder mehrwertige Alkohole, wie insbesondere Ethanol, Glycerin und Mischungen davon mit Wasser, wie z.B. 1 bis 50 Vol.-% Ethanol in Wasser.

Die Herstellung erfindungsgemäßer Darreichungsformen bzw. pharmazeutischer Mittel erfolgt unter Anwendung allgemein bekannter Methoden der pharmazeutischen Technologie, wie z.B. beschrieben in Voigt, Pharmazeutische Technologie, 7. Auflage 1993, Ullstein Mosby, Berlin.

In einer bevorzugten Ausführungsform wird ein pharmazeutisches Mittel bereitgestellt, das eine feste Dosierungsform umfasst. Diese feste Dosierungsform umfasst ihrerseits einen wirkstoffhaltigen festen Kern mit einem pharmazeutisch verträglichen Träger und einem Wirkstoffgehalt von etwa 1 bis 20 Gew.-%, bezogen auf das Gesamtgewicht des Kerns, wobei der Wirkstoff die Hydroxystilben-haltige Wirkstoffkombination gemäß den beiliegenden Ansprüchen ist.

Diese feste Dosierungsform hat beispielsweise einem Gesamt-Wirkstoffgehalt von etwa 1 bis 20 mg, wie z. B. 2 bis 10 mg, je Dosiseinheit und kann in Form einer Pille, einer Tablette, eines Extrudats oder eines Granulat vorliegen und z.B. dragiert sein. Gewünschtenfalls kann sie auch einen magensaftresistenten Überzug aufweisen.

Die feste Dosierungsform wird z.B. hergestellt, indem man die Wirkstoffkombination mit dem pharmazeutisch verträglichen Träger mischt und die Mischung zum Wirkstoffkem verfestigt. Dabei löst oder dispergiert man die Wirkstoffkombination in einer inerten Flüssigkeit, mischt ihn/sie mit dem Träger und entfernt das Lösungsmittel während oder nach der Verfestigung. Den Wirkstoffkem kann man dann gegebenenfalls mit einem magensaftresistenten Überzug versehen, bevor man den Kern in üblicher Weise dragiert.

Erfindungsgemäße flüssige Dosierungsformen werden z.B. dadurch hergestellt, indem man die Wirkstoffe, wie z.B. einen ERr731^{®} Trockenextrakt, in einem geeigneten Lösungsmittel, wie. z.B einem Wasser/Alkoholgemisch, ggf. zusammen mit weiteren üblichen Zusätzen, löst. Wirkstoffgehalte von 0,1 bis 20 oder 1 bis 10 mg/ml werden dabei gewöhnlich eingestellt.

Erfindungsgemäße halbfeste Dosierungsformen, wie z.B. Gele stellt man beispielsweise her, indem man die Wirkstoffe, wie z.B. einen ERr 731^{®} Trockenextrakt, in einem geeigneten Lösungsmittel, wie z.B einem Wasser/Alkoholgemisch, Alkohol oder Glycerin, löst und die Lösung in den bereits gequollenen Gelbildner, ggf. zusammen mit weiteren üblichen Zusätzen einarbeitet. Wirkstoffgehalte von 1 bis 12 oder 2 bis 6 mg pro Gramm der Formulierung werden dabei gewöhnlich eingestellt.

Art und Dauer der Verabreichung der erfindungsgemäßen Arzneimittel obliegen der Entscheidung des behandelnden Arztes. Dieser kann in Abhängigkeit vom gewählten Verabreichungsweg, von der Wirksamkeit der konkreten Wirkstoffzusammensetzung, der Art und Schwere der zu behandelnden Erkrankung, vom Befinden des Patienten und von dessen Ansprechen auf die Therapie eine geeignete Dosis und einen entsprechenden Dosierungsplan festlegen. Beispielsweise kann eine geeignete Einzeldosis etwa 0,1 bis 50 mg, wie z.B. 2 bis 12 mg, Wirkstoffkombination gemäß obiger Definition enthalten und 1 bis 3-mal täglich verabreicht werden, bis der gewünschte Behandlungserfolg zu beobachten ist.

### 2.2 Nahrungsergänzungsmittel und Lebensmittel

Zu den erfindungsgemäßen Mitteln gehören insbesondere auch Nahrungsergänzungsmittel und Lebensmittel, insbesondere funktionale oder diätetische Lebensmittel. Die erfindungsgemäßen Lebensmittel besitzen neben einer vorwiegend nährwertbezogenen Funktion zusätzlich eine wirkstoffbezogene Funktion, welche insbesondere die erfindungsgemäße Wirkstoffkombination betrifft. Sie werden daher als funktionale oder diätetische Lebens- oder Nahrungsmittel bezeichnet. Nahrungsergänzungsmittel dienen zur Ergänzung der täglichen Ernährung mit der erfindungsgemäßen Wirkstoffkombination, wobei die nährwertbezogene Funktion des Nahrungsergänzungsmittels für sich genommen in den Hintergrund tritt.

Die Formulierungsgrundlage für erfindungsgemäße Nahrungsergänzungsmittel und Lebensmittel umfasst ebenfalls physiologisch akzeptable Hilfsstoffe im weitesten Sinn, wie z.B. oben genannte Exzipienten. Hilfsstoffe im erfindungsgemäßen Sinne können auch einen Nährwert besitzen und deshalb allgemein als Nahrungskomponente verwendet werden. Auch Nährstoffe, insbesondere essentielle Nährstoffe, können dazu gehören.

Nahrungskomponenten enthalten in der Regel eine oder mehrere Aminosäuren, Kohlenhydrate oder Fette und sind für die menschliche und/oder tierische Ernährung geeignet. Sie umfassen Einzelkomponenten, häufig pflanzliche aber auch tierische Produkte, insbesondere Zucker gegebenenfalls in Form von Sirupen, Fruchtzubereitungen, wie Fruchtsäfte, Nektar, Fruchtpulpen, Pürees oder getrocknete Früchte, beispielsweise Apfelsaft, Grapefruitsaft, Orangensaft, Apfelmus, Tomatensauce, Tomatensaft, Tomatenpüree; Getreideprodukte, wie Weizenmehl, Roggenmehl, Hafermehl, Maismehl, Gerstenmehl, Dinkelmehl, Maissirup, sowie Stärken der genannten Getreide; Milchprodukte, wie Milcheiweiß, Molke, Joghurt, Lecithin und Milchzucker. Beispiele essentieller Nährstoffe sind insbesondere Vitamine, Provitamine, Mineralstoffe, Spurenelemente, Aminosäuren und Fettsäuren. Als essentielle Aminosäuren seien genannt Isoleucin, Leucin, Lysin, Methionin, Phenylalanin, Threonin, Tryptophan und Valin. Dazu gehören auch semi-essentielle Aminosäuren, die beispielsweise in Wachstumsphasen oder Mangelzuständen zugeführt werden müssen, wie Arginin, Histidin, Cystein und Tyrosin. Als Spurenelemente seien genannt: essentielle Spurenelemente und Mineralstoffe, wie: Eisen, Kupfer, Zink, Chrom, Selen, Calcium, Magnesium, Natrium, Kalium, Mangan, Cobalt, Molybdän, Iod, Silicium, Fluor, Chlor, Phosphor, Zinn, Nickel, Vanadium, Arsen, Lithium, Blei, Bor. Als für den Menschen essentielle Fettsäuren seien genannt: Linolsäure und Linolensäure, ARA (Arachidonsäure) und DHA (Docosahexaensäure) für Säuglinge und möglicherweise EPA (Eicosapentaensäure) und DHA auch für Erwachsene. Eine umfassende Aufzählung von Vitaminen findet sich in "Referenzwerte für die Nährstoffzufuhr", 1. Auflage, Umschau Braus Verlag, Frankfurt am Main, 2000, herausgegeben von der Deutschen Gesellschaft für Ernährung.

Beispiele geeigneter Formulierungen zur Nahrungsergänzung sind Kapseln, Tabletten, Pillen, Pulverbeutel, Flüssigampullen und Fläschchen mit Tropfeinsätzen, sowie die oben bereits genannten Arzneiformen.

Lebensmittel-Formufierungen haben in der Regel die übliche Form und werden beispielsweise als Frühstückszubereitungen, in Form von Müslis oder Riegeln, Sportlerdrinks, Komplettmahlzeiten, diätetische Zubereitungen, wie Diätdrinks, Diätmahlzeiten und Diätriegel, angeboten.

Die Herstellung erfindungsgemäßer Nahrungsergänzungsmittel und Lebensmittel erfolgt nach dem Fachmann geläufigen Methoden und bedarf keiner weiteren Erläuterung. ( vgl. z.B. Hans-Dieter Belitz et al. Lehrbuch der Lebensmittelchemie. Springer-Lehrbuch 5., überarb. Aufl. 2001. XLIV, 1059 Verlag: SPRINGER, BERLIN)

Der Gehalt an erfindungsgemäßen Wirkstoffkombinationen in obigen Nahrungsergänzungsmitteln und Lebensmitteln kann über einen weiten Bereich variieren und liegt z. B in einem Bereich von 0,01 bis 10 Gew.-%, wie z.B. 0,1 bis 1 Gew.-%.

### 2.3 Herstellung eines erfindungsgemäß verwendbaren Drogenextrakts

Erfingdungsgemäß verwendbare Drogenextrakte werden bevorzugt hergestellt, indem man
a) einen Hydroxystilben-haltigen Teil einer Drogenpflanze, gegebenenfalls in zerkleinerter Form, bereitstellt,
b) diesen mit einem wässrigen, organischen oder wässrig-organischen Extraktionsmittel versetzt,
c) nach Einwirken des Extraktionsmittels eine Flüssigextraktphase aus der Mischung gewinnt und die Extraktion gegebenenfalls mehrfach wiederholt, und
d) das Extraktionsmittel aus den so erhaltenen Flüssigextraktphasen entfernt.

Insbesondere umfasst der so erhaltene Extrakt die Wirkstoffkombination gemäß Anspruch 1.

Vorzugsweise liegen die extrahierten Hydroxystilbene aber im wesentlichen in der trans-Form vor.

In einer weiteren bevorzugten Ausführungsform des erfindungsgemäßen Verfahrens wird ein Extrakt bereitgestellt, der einen hohen Anteil an Glycosiden, insbesondere Glycosiden des oben beschriebenen Typs, aufweist, wie z.B. einen Anteil von 30 bis 100 Gew.-%, 50 bis 100 Gew.-%, 60 bis 99 Gew.-% oder 80 bis 98 Gew.-% oder 85 bis 96 Gew.-%, jeweils bezogen auf das Gesamtgewicht des erhaltenen Trockenextrakts. Ein "Trockenextrakt" in Sinne der Erfindung liegt insbesondere dann vor, wenn der Restfeuchtegehalt, d.h. der Restanteil an Wasser und/oder organischer Flüssigkeit, (wie Extraktionsmittel) weniger als etwa 5 Gew.-%, insbesondere weniger als 2 Gew.%, wie z.B. 0 bis 1,5 Gew.-% oder 0,1 bis 0,5 Gew.-%, jeweils bezogen auf das Gesamtgewicht des erhaltenen Trockenextrakts, beträgt.

In einer weiteren bevorzugten Ausführungsform wird ein Extrakt bereitgestellt, der im wesentlichen frei ist von Aglycon-Derivaten von Rhaponticin und Desoxyrhaponticin, wie insbesondere Resveratrol und Piceatannol. "Im Wesentlichen frei" bedeutet einen Aglycon-Gehalt von weniger als 5 Gew.-%, insbesondere weniger als 2 Gew.-% wie z.B. weniger als 1 Gew.-% oder 0,1 Gew.-%, wie 0 bis 0,05 Gew.-%, jeweils bezogen auf den Gesamtgehalt an Rhaponticin und Desoxyrhaponticin.

Weiterhin werden Wirkstoffkombinationen bevorzugt hergestellt, die einen Hydroxystilben-Gesamtgehalt von mehr als 90 Gew.-%, wie z.B. 91 bis 100 Gew.-%, oder 92 bis 99 oder 93 bis 98 oder 94 bis 97 Gew.-%, aufweisen.

Weiterhin werden solche Wirkstoffkombinationen bevorzugt hergestellt, die einen Gehalt von weniger als 0,5 Gew.-% , wie z.B. 0 - 0,49 Gew.% oder 0,001 bis 0,3 oder 0,01 bis 0,2 oder 0,01 bis 0,1Gew.-% an Anthrachinon und/oder Anthrachinoiden (jeweils bezogen auf Trockengewicht der Wirkstoffkombination) aufweisen. Anthrachinoide sind dabei im weitesten Sinn als Substanzen mit Anthrachinon-Grundgerüst zu verstehen.

In einer bevorzugten Ausführungsform ist die zu extrahierende Drogenpflanze ausgewählt unter natürlichen Pflanzen sowie durch Züchtung veränderten oder rekombinanten, genetisch veränderten, Pflanzen, die einen in Vergleich zur entsprechenden unveränderten Pflanze höheren Gehalt an wenigstens einem der gewünschten Inhaltsstoffe aufweisen. Insbesondere sind diese Pflanzen ausgewählt unter Pflanzen der Gattung Rheum spp., Astragalus spp., Cassia spp. oder Picea spp. oder wirkstoffhaltigen Pflanzenteilen. Nichtlimitierende Beispiele für geeignete Arten dieser Gattungen sind Rheum undulatum, Rheum palmatum, Rheum tataricum, Rheum officinale, Rheum wittrockii, Rheum altaicum, Rheum reticulatum, Astragalus complanatus, Cassia garrettiana und Picea sitchensis. Bevorzugt ist außerdem der Einsatz sortenreiner Drogenpflanzen.

Dem Fachmann ist außerdem bewusst, dass Gattungen/Arten unterschiedlicher Provinienz sowie verschiedenen Alters (d.h. Ernte zu verschiedenen Zeiten der Vegetationsperiode) einsetzbar sind, was wiederum Einfluss auf Art, Menge und Zusammensetzung der daraus isolierbaren Hydroxystilbene und Gemische haben kann. Ebenso sind verschiedene Pflanzenteile, wie Wurzeln, Rhizome, Blätter und/oder Stängel, grundsätzlich brauchbar.

Das jeweilige Pflanzenteil oder Gemisch von Pflanzenteilen kann, falls zweckmäßig, vor der Extraktion mechanisch behandelt, wie z.B. gemahlen, gehackt, gehaspelt, gestampft oder geschnitten werden. Falls zweckmäßig, kann auch eine Vortrocknung, wie z.B. 2 Stunden bis 2 Tage bei 30 bis 50°C erfolgen, um den Flüssigkeitsgehalt zu reduzieren.

Insbesondere ist der zur Extraktion verwendete, Hydroxystilben-haltige Teil der Drogenpflanze die Wurzel der Drogenpflanze, wie z.B. von Rheum rhaponticum.

Insbesondere geeignet ist ein Verfahren, bei dem man von der Droge ein Hydroxystilben-haltiges Perkolat herstellt. Unter einer "Perkolation" versteht man ein kontinuierliches Ausziehen löslicher Stoffe einer Droge durch laufende Erneuerung des Lösungsmittels. Dadurch besteht dauernd ein Konzentrationsgefälle, so dass ein großer Teil aller löslichen Stoffe in den Auszug übergeht.

Alternativ ist auch eine kontinuierliche oder periodische Durchmischung des Ansatzes, wie z.B. durch Rühren oder Schütteln, möglich.

Die Temperatur während der erfindungsgemäßen Extraktion liegt gewöhnlich im Bereich von 10 bis 50°C, wie z.B. 25 bis 35°C. Der Druck liegt gewöhnlich bei Normaldruck. Falls eine Beschleunigung der Extraktionsgeschwindigkeit oder Extraktqualität erzielbar ist, kann der Druck während der Extraktion auch variiert, wie z.B. erhöht oder verringert werden.

Die Extraktionsdauer kann dabei je nach den gewählten Bedingungen, wie Art der Droge, Ansatzgröße, verwendetem Extraktionsmittel und Temperatur, 1 Stunde bis mehrere Tage, wie z.B. 10 bis 72 Stunden erfordern.

Der Extraktionsvorgang kann erforderlichenfalls mehrfach wiederholt werden, um eine möglichst vollständige Isolierung insbesondere der gewünschten Inhaltsstoffe zu gewährleisten. Das Gewichtsverhältnis von vorgelegter Droge zu flüssigem Extraktionsmittel kann über einen weiten Bereich sowie von Extraktionsschritt zu Extraktionsschritt variieren. Typischerweise liegt das Gewichtsverhältnis von Droge zu Extraktionsmittel im Bereich von 10:1 bis etwa 1:200 oder etwa 1:2 bis 1:50, oder 1:4 bis 1:10.

Gemäß einer Verfahrensvariante führt man eine Extraktion mit einem wässrigen, im wesentlichen an organischem Lösungsmittel freiem Extraktionsmittel, wie insbesondere Wasser, vorzugsweise gereinigtem Wasser, bei einem pH-Wert der Mischung im alkalischen Bereich durch, wobei der pH-Wert der Mischung insbesondere im Bereich von etwa 11 bis 12, wie z.B. bei etwa 11,3 bis 11,8 liegt.

Dabei wird der pH-Wert der Mischung beispielsweise mit Hilfe einer anorganischen Base, ausgewählt unter Alkali- und Erdalkalimetallhydroxiden, wie z.B. Calciumhydroxid oder Calciumoxid, eingestellt. Zu diesem Zweck kann man beispielsweise eine konzentrierte Löschkalklösung herstellen, indem man 3 bis 8 Teile CaO in 20 Teilen gereinigtem Wasser löst. Diese Lösung ist stark alkalisch und weist einem pH-Wert im Bereich von etwa 12 bis 13, wie z.B. von etwa 12,4 bis 12,6, auf.

Das Mengenverhältnis von vorgelegter Droge zu Base, wie z.B. Calciumhydroxid (berechnet als Calciumoxid) kann erfindungsgemäß im Bereich von etwa 5:1 bis 20:1, wie etwa 8:1 bis 12:1 oder 9:1 bis 11:1, liegen.

Bevorzugt wird das Verfahren so durchgeführt, dass man aus der erhaltenenen alkalischen Flüssigextraktphase die gewünschten Hydroxystilbene ausfällt, beispielsweise indem man den pH-Wert des Extrakts auf einen Wert im Bereich von etwa 3 bis 4, wie z.B. 3,2 bis 3,8 oder 3,4-3,6, einstellt und gegebenenfalls anschließend den Niederschlag abtrennt, gegebenenfalls wäscht und gegebenenfalls trocknet.

Zur Ansäuerung verwendet man dabei eine beliebige anorganische oder organische Säure, wie z.B. Salzsäure oder Schwefelsäure, insbesondere aber organische Säuren wie Ameisensäure oder Essigsäure.

Vor dem Abtrennen des Niederschlags kann es zweckmäßig sein, den Ansatz einige Stunden oder Tage ruhen zu lassen, um eine möglichst quantitative Fällung der gewünschten extrahierten Inhaltsstoffe zu erreichen.

Das Waschen des Niederschlags kann z.B. mit gereinigtem Wasser erfolgen und dient insbesondere zur Entfernung restlicher Säure.

Durch Trocknen, z.B. bei 30 bis 50°C oder 35 bis 45°C, beispielsweise über einen Zeitraum von 1 bis 100 Stunden, wird restliche Flüssigkeit aus dem Extrakt entfernt, bis die Restfeuchte im oben angegebenen Bereich liegt. Die Trocknung erfolgt in an sich bekannter Weise, z.B. in einem Trockenschrank. Eine Gefriertrocknung ist ebenfalls möglich.

Die Erfindung wird nun anhand folgender nichtlimitierender Beispiele und unter Bezugnahme auf beiliegende Figuren weiter erläutert.

### EXPERIMENTELLER TEIL

### Allgemeine Methoden:

### Bestimmung von Stilbenen durch Hochdruckflüssigkeitschromatographie (HPLC) in Trockenextrakt aus Rhapontikrhabarberwurzel

### a) Probenvorbereitung:

50 mg Extrakt werden mit 40 ml einer Mischung aus Aceton und Wasser (1:1) in einem Braunglasgefäß versetzt, 15 Minuten im Ultraschallbad behandelt und mit der Lösungsmittelmischung auf 50 ml aufgefüllt und anschließend 1:10 mit der Lösungsmittelmischung verdünnt.

### b) Durchführung der Chromatographie:

Mit einem Teil der oben erhaltenen Lösung wird eine Hochdruckflüssigkeitschromatographie (HPLC) mit folgenden Systemparametern durchgeführt:

| | |
|---|---|
| Probenschleife: | 20µl |
| Säule: | Lichrospher 5 µ RP 18, 250 x 4 mm |
| Vorsäule: | Lichrospher 5 µ RP 18, 5 x 4 mm |
| Säulentemperatur: | 25 °C |
| Fließmittel A: | Acetonitril/dest. Wasser/Phosphorsäure 85 %, 15/85/0,05 (Volumenteile) |
| Fließmittel B: | Acetonitril/dest. Wasser/Phosphorsäure 85 % 80/20/0,05 (Volumenteile) |
| Fluss: | 1,5 ml/min |
| Säulenspülung: | 15 min mit Eluent 50 % B; Equilibrierzeit 15 min |
| Detektion: | 310 nm |
| HPLC: | Kontron Kroma 2000 |

### Gradient:

| **Zeit** | **% B** |
|---|---|
| 0,0 | 0 |
| 0,5 | 0 |
| 7,5 | 75 |
| 8,5 | 100 |
| 9,5 | 0 |
| 12,5 | 0 |

Unter den oben angegebenen Systembedingungen ergeben sich folgende Retentionszeiten:

| | |
|---|---|
| Rhaponticin: | ca. 5,5 min |
| Desoxyrhaponticin: | ca. 6,8 min |
| Rhapontigenin: | ca. 7,2 min |
| Desoxyrhapontigenin: | ca. 9,0 min |

Für eine quantitative Bestimmung werden die jeweiligen Peak-Flächen ermittelt und mit den entsprechenden Peak-Flächen eines Standard-Extraktes bekannter Zusammensetzung verglichen.

### Herstellungsbeispiel 1: Herstellung des Trockenextraktes ERr 731 aus Rhapontikrhabarberwurzel mit einer wässrigen Calciumhydroxid-Lösung

Für die Herstellung eines Trockenextraktes aus Rhapontikrhabarberwurzel werden eingesetzt:

| | |
|---|---|
| Droge (Radix rheum rhaponticum) | 50,0 kg |
| Calciumoxid | 5,0 kg |
| Gereinigtes Wasser | 190,0 kg |

Essigsäure (je nach Bedarf um den erforderlichen pH-Wert einzustellen)

Die erzielbare Ausbeute liegt dabei zwischen 2 und 3 kg je 50 kg Droge.

Die Herstellung erfolgt in folgenden Schritten:
a) In einen Plastikbottich werden zunächst 5 kg Calciumoxid gefüllt und mit 20 kg gereinigtem Wasser aufgeschlämmt. Die unter diesen Bedingungen stattfindende Bildung von Calciumhydroxid (Löschkalk) führt zu einem starken Temperaturanstieg der Lösung. Daher kann das Calciumhydroxid erst nach Abkühlen weiter verwendet werden. Die Temperatur der Lösung liegt dann bei 30°C bis 35°C.
b) 50 kg Droge werden in einen Mischer gefüllt und mit dem oben erwähnten Löschkalk versetzt. Um den Löschkalk möglichst vollständig aus dem Plastikbottich zu entfernen, wird dieser mit 10 kg gereinigtem Wasser nachgespült. Diese Waschflüssigkeit wird ebenfalls in den Mischer gegeben.
c) Die mit Löschkalk homogen durchmischte Droge wird in einen Perkolator gefüllt und mit 160 kg gereinigtem Wasser überschichtet. Der Perkolator bleibt für 48 Stunden verschlossen. Anschließend wird das Perkolat in einem geeigneten Gefäß mit einer Flussrate von 50ml/min aufgefangen. Die Perkolation wird solange fortgesetzt, bis kein Perkolat mehr austritt. Die Drogenmasse wird nach Beendigung nicht ausgepresst, sondern verworfen.
d) Unter permanenter Kontrolle wird dem Perkolat solange konzentrierte Essigsäure zugefügt, bis ein pH-Wert erreicht ist, der im Bereich von 3,4 bis 3,6 liegt. Um eine möglichst vollständige Fällung des Extraktes zu erreichen, ruht der Ansatz für 5 Tage.
e) Die Gewinnung des Trockenextraktes erfolgt durch Filtration über Büchnertrichter unter angelegtem Vakuum. Abschließend wird der Extrakt noch mit 10 bis 20 kg gereinigtem Wasser gewaschen.
f) Der nach Filtration erhaltene Trockenextrakt wird im Trockenschrank bei 40°C solange getrocknet, bis eine Restfeuchte-Toleranz von max. 1 % erreicht ist.

Rhaponticin ist in wässrigen Lösungen mit alkalischem pH-Bereich gut löslich, während es im sauren pH-Bereich (pH 3,4 - 3,6) als gelbliche Substanz ausfällt. Dies macht man sich bei seiner Isolierung zu Nutzen. Da die Wurzel neben anderen organischen Säuren vor allem einen hohen Gehalt an Oxalsäure besitzt (2/3 in wasserlöslicher und 1/3 in gebundener Form) muss diese während der Isolation neutralisiert werden, um ein Abdriften des pH-Werts in den sauren Bereich zu verhindern und so ein frühzeitiges Ausfallen des Rhaponticins zu unterbinden. Dies wird durch den Einsatz von Calciumoxid erreicht. Dieses wird als Löschkalk-Lösung mit einem pH-Wert von 12,4 - 12,6 eingesetzt.

Wird der Löschkalk mit der Droge homogen durchmischt, so verändert sich der pH-Wert der Mischung. Er liegt dann im Bereich von 11,3 bis 11,8, wodurch ein Ausfällen von Rhaponticin verhindert wird, da die phenolische Form in eine Phenolat-Form überführt wurde. Trotz des hohen Gehalts an Oxalsäure kann der pH-Wert im alkalischen Bereich gehalten werden. Dies ist darauf zurückzuführen, dass das Calciumhydroxid mit Oxalsäure reagiert und unlösliches und ungiftiges Calciumoxalat bildet.

Durch die anschließende Perkolation mit gereinigtem Wasser wird Rhaponticin aus der Wurzel extrahiert. Nach Beendigung der Perkolation wird durch Zugabe von Essigsäure ein pH-Wert von 3,4 bis 3,6 eingestellt. Dieser pH-Shift vom alkalischen in den sauren Bereich führt zum Ausfällen von Rhaponticin durch Rückführung in die phenolische Form. Um eine möglichst vollständige Fällung des Rhaponticins zu erreichen, lässt man den Ansatz 5 Tage stehen. Danach wird abfiltriert. Rhaponticin bleibt als gelbliche Substanz auf dem Filter zurück.

Obige Ausführungen zu Rhaponticin gelten entsprechend für die anderen erfindungsgemäß isolierten Hydroxystilbenwirkstoffe.

### Herstellungsbeispiel 2: Herstellung eines Trockenextraktes aus Rhapontikrhabarberwurzel mit verschiedenen organischen Lösungsmitteln

In der hier als Droge verwendeten Rhapontikrhabarberwurzel gehören die hauptsächlich nachweisbaren Inhaltstoffe zur Gruppe der Hydroxystilbene. Aus dieser Gruppe finden sich in den Wurzeln Rhaponticin (Rh) mit einem Anteil von etwa 6 % und Desoxyrhaponticin (DRh) mit einem Anteil von etwa 4 %.

Bei Einwirkung der im Folgenden angegebenen Lösungsmittelsysteme in der 100-fachen Menge, bei Raumtemperatur für 10 Minuten unter Schütteln oder Rühren lassen sich die nachfolgend zusammengefassten Anteile extrahieren:

| | | |
|---|---|---|
| **Ethanol 86 %** | Rh | 100,8 % |
| | DRh | 99,5 % |
| | | |
| **Ethanol 15 %** | Rh | 77,1 % |
| | DRh | 75,5 % |
| | | |
| **Aceton** | Rh | 88,3 % |
| | DRh | 96,6 % |
| | | |
| **Wasser, alkalisch** | Rh | 75,5 % |
| (pH 11, eingestellt mit CaO-Lösung) | DRh | 60,5 % |

Keine brauchbaren Resultate wurden mit Heptan erzielt.

Die jeweiligen Ausbeuten an Rohextrakten in Massenanteilen (bezogen auf eingesetzte Droge) sind folgende:

| | |
|---|---|
| **Ethanol 86 %** | 35,5 % |
| **Ethanol 15 %** | 32,2 % |
| **Aceton** | 21,4 % |
| **Heptan** | 0 % |
| **Wasser, alkalisch** | 4,5 % |

Die Extraktion von Rhapontikrhabarberwurzel mit Ethanol-Wasser-Gemischen führt zu einem Extrakt, der neben den Hauptinhaltsstoffen Rhaponticin (ca. 30 %) und Desoxyrhaponticin (ca. 22 %), ein weiteres z.Zt. noch nicht erforschtes Stilben in ca. 20 % Anteil am Extrakt enthält. Daneben erhält man die Aglycone Rhapontigenin (ca. 8 %) und Desoxyrhapontigenin (ca. 2 %) und noch 9 weitere Verbindungen, die sich zu ca. 20 % summieren.

Bei der Extraktion mit Aceton erhält man grundsätzlich gleiche Ergebnisse.

Die Extraktion mit alkalisiertem Wasser (vgl. Bedingungen gemäß Herstellungsbeispiel 1) führt zu einem Extrakt höherer Reinheit.

Die Hauptinhaltsstoffe Rhaponticin und Desoxyrhaponticin sind in einen ca. 97 %igen Anteil im trockenen Extrakt enthalten. Rhapontigenin und Desoxyrhapontigenin ergeben im Extrakt einen Anteil von zusammen 1,1 %, während das noch nicht erforschte Stilben lediglich in einem Anteil von 0,2 % enthalten ist. Weitere 3 Verbindungen sind in einem 2,5 %igen Anteil enthalten.

### Formulierungsbeispiel 1: Herstellung einer festen Dosierungsform -Minitablette

### 1. Herstellung des Tablettenkerns:

Ein fester Tablettenkern wird unter Verwendung folgender Wirk- und Hilfsstoffe in den angegebenen Mengenverhältnissen (TI = Gewichtsteile) hergestellt. Die Bestandteile werden auf drei verschieden Weisen vermischt und tablettiert:

### a) Rezeptur für Tablettenkern:

| | | |
|---|---|---|
| Gereinigter Trockenextrakt gemäß | | |
| Herstellungsbeispiel 1 | | |
| aus Rhapontikrhabarberwurzel (ERr 731^{®}) | 3,6 Tl | |
| Mikrokristalline Cellulose (z.B. Avicel^{®}) | 57,0 Tl | (± 40 %) |
| Sorbit | 8,0 Tl | " |
| Talkum | 2,5 Tl | " |
| Makrogol 6000 (Polyglykol) | 1,6 Tl | " |
| Polyvidon (K-Wert ca. 25, z.B. Kollidon^{®} 25) | 1,6 Tl | " |
| Natriumdodecylsulfat (z.B. Texapon^{®} K 12) | 0,5 Tl | " |
| Magnesiumstearat (pflanzlich) | 0,8 Tl | " |
| | 75,6 TI (± 40 %) | |

Durch Variation der eingewogenen ERr 731®-Menge und/oder Variation der mikrokristallinen Cellulose-Menge können beliebige ERr 731®-Gehalte des Rohkerns erhalten werden (wie z.B. 2, 4, 6, 8, 10, 12 mg je Tablette).

### b) Mischen von Droge und Träger

### - Mischungs-Variante a:

1,2 Tl ERr® 731 werden anteilig mit Avicel^{®} in der Kugelmühle verrieben, anschließend wird nach Zugabe der übrigen Hilfsstoffe wie unten beschrieben gemischt und tablettiert.

### - Mischungs-Variante b:

ERr 731 (1g/l Lösungsmittel) wird in einem geeigneten Lösungsmittel (z.B. Ethanol/Wassergemisch 86% v/v Ethanol) gelöst und auf Avicel^{®} aufgezogen, getrocknet (bei 40°C mindestens 48 Stunden) und nach Zugabe der übrigen Hilfsstoffe wie unten beschrieben gemischt und tablettiert.

### - Mischungs-Variante c:

Die gesamte Avicel®-Menge wird in drei gleiche Portionen aufgeteilt. Die erste Portion wird mit der Gesamtmenge ERr731® versetzt und in einer Laborkugelmühle (z.B. Typ 1-25 LK, Alpine, Augsburg) mindestens für 120 Minuten verrieben. Dann gibt man die zweite Portion Avicel® hinzu, verreibt erneut mindestens 120 Minuten in der Laborkugelmühl. Nach Zugabe der dritten Portion Avicel® wird nochmals kurz vermischt. Anschließend wird nach Zugabe der übrigen Hilfsstoffe wie unten beschrieben gemischt und tablettiert.

Mit dieser Mischungsvariante gelingt es überraschenderweise, die Entmischungsneigung deutlich zu verringern und selbst bei kleinen Dosiseinheiten einen äußerst gleichförmigen Wirkstoffgehalt von nicht mehr als ± 5 Gew.-% (bestimmt nach Ph. Eur. 5. Ausgabe 2005 (5.0/2.09.06.00)) einzustellen.

### c) Tablettierung:

Die Mischung aus Avicel® und Wirkstoff wird durch eine Siebmaschine (Siebdurchmesser 1.2 mm) in einen geeigneten Mischbehälter gesiebt und nach Zugabe der angegebenen Tablettierungshilfsmittel (ohne Magnesiumstearat) in einem geeigneten Mischer (z.B. Rhönrad-Mischer Typ Standard RR M 200, Firma Engelsmann AG/Ludwigshafen) mind. 30 min gemischt. Nach Zugabe von Magnesiumstearat wird nochmals mind. 5 min gemischt.

Die Verpressung erfolgt auf einer geeigneten Tablettenpresse (z.B. Rundläufer Typ Perfecta Fette 2000, Fa. Fette/Schwarzenbeck):

| | |
|---|---|
| Kerngewicht: | 84 mg ± 4.2 mg maximale Abweichung |
| Stempel: | 7 mm Durchmesser, drageegewölbt |

Der ERr-731-Gehalt je Kern beträgt etwa 4 mg ± 5%.

### 2. Herstellung der magensaftresistenten überzogenen Tablette

Nach dem Entstauben der Tablettenkerne mit Eudragit wird ein magensaftresistenter Überzug aus Celluloseacetatphthalat und Diethylphthalat, gelöst in Isopropanol und Ethylacetat, auf die Tablettenkerne mit Hilfe einer Coatinganlage aufgebracht. Macrogol wird in gereinigtem Wasser gelöst. Die Bestandteile Zucker (Saccharose oder Isomalt), Calciumcarbonat, Talkum, Titaniumdioxid und die beiden Povidone werden gemischt und in die Flüssigkeit eingerührt. Die Suspension wird 20 Minuten im Leitstrahlmischer (z.B. Rototron Typ RTA 70-50) gerührt.

Die Dragiersuspension wird mit Hilfe eines Dragierautomaten auf die isolierten Kerne aufgebracht. Der Vorgang wird so lange wiederholt, bis ein durchschnittliches Gewicht von 150 mg pro aufdragiertem Kern erreicht ist. Schließlich trägt man das Polierwachs auf und anschließend lässt man bis zum Hochglanz rollieren.

Endgewicht der magensaftresistenten überzogenen Tablette:
150 mg ± 7.5 mg maximale Abweichung.

Auf diese Weise werden zwei verschieden Tablettenformen - eine zuckerhaltige und eine zuckerfreie - hergestellt, wobei die jeweiligen Hilfsstoffen in den im Folgenden angegebenen Gewichtsteilen eingesetzt wurden.

### a) Magensaftresistente überzogene Minitablette - zuckerhaltig - mit Weichmacher im Coating

### Hilfstoffe:

| | | | |
|---|---|---|---|
| Coating: | Eudragit L12,5%Trockensubstanz | 1,350 kg | (±40 %) |
| | Diethylphthalat | 1,749 kg | " |
| | Celluloseacetatphthalat | 7,770 kg | " |
| | Isopropylalkohol | 75,600 kg | " |
| | Ethylacetat | 77,600 kg | " |
| | Talkum | 2,000 kg | " |
| | | | |
| Dragierung: | Talkum | 7,182 kg | " |
| | Zucker | 28,747 kg | " |
| | Calciumcarbonat | 6,410 kg | " |
| | Titandioxid E 171 | 0,635 kg | " |
| | Povidon | | |
| | (K-Wert ca. 25, z.B. Kollidon^{e} 25) | 0,756 kg | " |
| | Povidon (K-Wert: ca. 90) | 0,332 kg | " |
| | Macrogol 35.000 | 0,635 kg | " |
| | Wasser | 10,500 kg | " |
| | | | |
| Politur: | 95 % Carnaubawachs, | | |
| | 5 % Gebleichtes Wachs | | |
| | (z.B. Capol 1295 PH) | 0,108 kg | " |

### b) Magensaftresistente überzogene Minitablette - zuckerfrei - mit Weichmacher im Coating

| | | | |
|---|---|---|---|
| Hilfstoffe: | | | |
| | | | |
| Coating: | Eudragit L12,5% Trockensubstanz | 1,350 kg | (± 40 %) |
| | Diethylphthalat | 1,749 kg | " |
| | Celluloseacetatphthalat | 7,770 kg | " |
| | Isopropylalkohol | 75,600 kg | " |
| | Ethylacetat | 77,600 kg " | " |
| | | | |
| Dragierung: | Talkum | 7,482 kg | " |
| | Sorbit und/oder Isomalt | 28,747 kg | " |
| | Calciumcarbonat | 6,410 kg | " |
| | Titandioxid E 171 | 0,635 kg | " |
| | Povidon (K-Wert ca. 25, z.B. Kollidon^{®} 25) | 0,756 kg | " |
| | Povidon (K-Wert: ca. 90) | 0,332 kg | " |
| | Macrogol 35.000 | 0,635 kg | " |
| | Wasser | 10, 500 kg | " |
| | | | |
| Politur: | 95 % Carnaubawachs, | | |
| | 5 % Gebleichtes Wachs | | |
| | (z.B. Capol 1295 PH) | 0,108 kg | " |

### Formulierungsbeispiel 2: Herstellung einer festen Dosierungsform - Minitablette zuckerhaltig ohne Weichmacher

### 1. Herstellung des Tablettenkems

Die Herstellung erfolgt analog Formulierungsbeispiel 1.

### 2. Herstellung der magensaftresistenten überzogenen Tablette

Die Herstellung erfolgt analog Formulierungsbeispiel 1, wobei jedoch anstelle von Cellulsoeacetatphthalat /Diethylphthalat (Weichmacher) Schellack (Variante A) oder Aqoat (Variante B) verwendet wird.

### a) Variante A

| Hilfsstoffe: | | kg (± 40 %) |
|---|---|---|
| Coating: | Eudragit L12,5% Trockensubstanz | 0,400 |
| | CAPOL 5270 PH 8% | |
| | (Schellacklösung) | 60,000 |
| | = 4,8 kg Trockensubstanz | |
| | (Schellack) | |
| | Isopropylalkohol | 4,000 |
| | Ethanol 96% | 3,200 |
| | Talkum | ..2,000 |
| | | |
| Dragierung: | Talkum | 7,182 |
| | Zucker | 28,747 |
| | Calciumcarbonat | 6,410 |
| | Titandioxid E 171 | 0,635 |
| | Polyvidon (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### b) Variante B

| Hilfsstoffe: | | kg (± 40 %) |
|---|---|---|
| Coating: | Eudragit L12,5% Trockensubstanz | 0,400 |
| | Aqoat Hydroxypropylmethylcelluloseacetatsuccinat | 5,420 |
| | Aqua dest. | 12,500 |
| | Isopropylalkohol | 4,000 |
| | Ethanol 86% | 55,000 |
| | | |
| Dragierung: | Talkum | 9,182 |
| | Zucker | 28,747 |
| | Calciumcarbonat | 6,410 |
| | Titanoxid E 171 | 0,635 |
| | Polyvidon (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### Formulierungsbeispiel 3: Herstellung einer festen Dosierungsform - Minitablette zuckerfrei ohne Weichmacher

### 1. Herstellung des Tablettenkerns

Die Herstellung erfolgt analog Formulierungsbeispiel 1, wobei jedoch anstelle von Avicel Isomalt verwendet wurde.

### 2. Herstellung der magensaftresistenten überzogenen Tablette

Die Herstellung erfolgt analog Formulierungsbeispiel 2, wobei jedoch anstelle von Zucker Isomalt verwendet wurde.

### a) Variante A

| | | |
|---|---|---|
| Hilfsstoffe: | | kg (± 40 %) |
| Coating: | Eudragit L12,5% Trockensubstanz | 0,400 |
| | CAPOL 5270 PH 8% | |
| | (Schellacklösung) | 60,000 |
| | = 4,8 kg Trockensubstanz | |
| | (Schellack) | |
| | Isopropylalkohol | 4,000 |
| | Ethanol 96% | 3,200 |
| | Talkum | 2,000 |
| | | |
| Dragierung: | Talkum | 7,182 |
| | Isomalt | 28,747 |
| | Calciumcarbonat | 6,410 |
| | Titanoxid E 171 | 0,635 |
| | Polyvidon (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### b) Variante B

| Hilfsstoffe: | | kg (± 40 %) |
|---|---|---|
| Coating: | Eudragit L12,5% Trockensubstanz | 0,400 |
| | Aqoat | 5,420 |
| | Aqua dest. | 12,500 |
| | Isopropylalkohol | 4,000 |
| | Ethanol 86% | 55,000 |
| | Talkum | ..2,000 |
| | | |
| Dragierung: | Talkum | 7,182 |
| | Isomalt | 28,747 |
| | Calciumcarbonat | 6,410 |
| | Titanoxid E 171 | 0,635 |
| | Polyvidon (K-Wert ca. 25, z.B. Kollidon^{®} 25) | 0,756 |
| | Povidon (K-Wert: ca. 90) | 0,332 |
| | Macrogol 35.000 | 0,635 |
| | Wasser | 10,500 |
| | | |
| Politur: | 95 % Carnaubawachs | |
| | 5 % Gebleichtes Wachs | |
| | (z.B. Capol 1295 PH) | 0,108 |

### Formulierungsbeispiel 4: Herstellung einer halbfesten Dosierungsform - Vaginalgel

Die Herstellung erfolgt unter Anwendung üblicher Methoden nach nach den beiden folgenden Varianten:

### a) Variante A:

Hydroxypropylmethylcellulose (Hypromellose USP) oder ein anderer Gelbildner wird mit 2-10 Gew.-% in gereinigtem Wasser quellen gelassen. Anschließend wird das in Glycerin gelöste ERr 731^{®} (Herstellungsbeispiel 1) eingearbeitet. Die Glycerinmenge kann bis zu 50 Gew.-% des Gels betragen. ERr 731^{®} kann in Glycerin bis zu 0,5 Gew.-% gelöst werden. Im Bedarfsfall können dem Gel Konservierungsstoffe (z. B. Sorbinsäure und ihre Salze) hinzugegeben werden. Auch eine pH-Einstellung ist möglich. Alternativ zu Glycerin kann auch Ethanol 30 - 86 Vol.% verwendet werden.

### b) Variante B:

Carbomer (Carbopol) wird mit 0,5 - 5 Gew.-% in gereinigtem Wasser gelöst und der gewünschte pH-Wert (z. B. KOH, NaOH, NH₃) eingestellt. Wenn notwendig, wird ein Konservierungsmittel (z. B. Sorbinsäure und ihre Salze) beigemischt. Nach Bildung eines klaren Gels wird ERr 731^{®} (Herstellungsbeispiel 1) bis zu 0,5 Gew.-% in Ethanol 30 - 86 Vol.-% gelöst und zugegeben. Alternativ zu Ethanol kann auch Glycerin verwendet werden.

### Formulierungsbeispiel 5: Herstellung einer halbfesten Dosierungsform - Vaginalkugeln

Es werden Kugeln mit einer Größe von 1 bis 15 g mit einem Gehalt von 1 bis 12 mg ERr 731^{®} (Herstellungsbeispiel 1) gelöst in Glycerin (85 % n 20/D =1,45085) nach zwei verschiedenen Varianten in herkömmlicher Weise hergestellt.

### a) Variante A:

### Rezeptur:

| | |
|---|---|
| Gelatine | 1 Teil |
| Gereinigtes Wasser | 2 Teile |
| Glycerin 85 % (+ERr 731^{®}) | 5 Teile |

### b) Variante B:

Gleiche Rezeptur, jedoch mit geeignetem Konservierungsmittel, wie z. B. Sorbat, Benzoat, PHB-Ester.

Die Gelatine wird jeweils in gereinigtes Wasser eingebracht und quellen gelassen bis alles glasig geworden ist. Anschließend wird Glycerin 85 % mit Wirkstoff dazugegeben und erwärmt, jedoch nicht über 65°C. Anschließend werden die Globuli in üblicher Weise gegossen

### Formulierungsbeispiel 6: Herstellung einer flüssigen Dosierungsform - Tropfen

### a) Lösungsversuche mit ERr 731^{®} in Ethanol und Glycerol:

Gehalt des Extrakts:
61,9 % Rhaponticin
29,9 % Desoxyrphaponticin

Versuch A: 200 mg Trockenextrakt in 50 ml Glycerol R:

| | |
|---|---|
| 55,1 % Rhaponticin | (89,0 % der Theorie) |
| 27,1 % Desoxyrhaponticin | (90,6 % der Theorie) |

Versuch B: 200 mg Trockenextrakt in 50 ml Ethanol 30 % R:

| | |
|---|---|
| 52.2 % Rhaponticin | (84,3 % der Theorie) |
| 25,2 % Desoxyrhaponticin | (84,2 % der Theorie) |

Versuch C: 200 mg Trockenextrakt in 50 ml Ethanol 50 % R:

| | |
|---|---|
| 58,8 % Rhaponticin | (95,0 % der Theorie) |
| 29,0 % Desoxyrhaponticin | (97,0 % der Theorie) |

Versuch D: 200 mg Trockenextrakt in 50 ml Ethanol 86 % R:

| | |
|---|---|
| 59,8 % Rhaponticin | (96,6 % der Theorie) |
| 29,5 % Desoxyrhaponticin | (98,7 % der Theorie) |

### b) Herstellung von Tropfen:

Zur Herstellung von Tropfen wurde Trockenextrakt gemäß Versuch B in Ethanol 30% R gelöst und gegebenenfalls filtriert.
Aglycon

### Testbeispiel 1: Pharmakokinetik und in vivo-Akkumulation und -Metabolisierung der Inhaltsstoffe von ERr 731^{®}

### a) Pharmakokinetik von ERr 731^{®} Inhaltsstoffen bei weiblichen Probanden

Nach oraler Einnahme von ERr 731^{®} sollte überprüft werden, ob sich einer der Inhaltsstoffe dieser Wirkstoffkombination im Blut wieder finden lässt, um zu beweisen, dass mindestens einer der Bestandteile dieser Wirkstoffkombination oder dessen Metabolite bioverfügbar ist.

Von einer Freiwilligen wurden morgens (8.00 Uhr) 10 Tabletten ERr 731^{®} (Dosierung = 40 mg ERr 731^{®}) mit Flüssigkeit eingenommen. Anschließend wurde zu verschiedenen Zeitpunkten (wie in Figur 1 angegeben) 10 ml Blut abgenommen und durch Abzentrifugieren das Plasma gewonnen.

Diese Plasmaproben wurden wie folgt aufgearbeitet: 500 pl Plasma wurden mit 25 µl einer internen Standardarbeitslösung (2.5 ng/µl Rhaponticin oder Rhapontigenin in Methanol) versetzt und mit 500 µl isotonischer NaCl-Lösung und 2.5 ml Diethylether/Butanol (9/1; v/v) gemischt. Nach Schütteln und Zentrifugieren (10 Minuten bei 4600 rpm), wurden ca. 2 ml des Überstandes abgenommen und unter Stickstoffbegasung (bei 60°C) getrocknet. Das Pellet wurde in 50 µl Methanol aufgenommen. Nach Zugabe von 200 µl destilliertem Wasser wurde erneut gemischt, und 200 µl wurden in Autosampier Röhrchen pipettiert (lichtgeschützt). 30 µl der Proben wurden in ein LC-MS/MS System (PE Sciex API 3000) zur Analyse injiziert. Die chromatographische Trennung der Analyte erfolgte auf einer Phenomenex Polymer X Säule mit einem Gradienten aus einer Ammoniumpufferlösung und einem Acetonitril/Methanol-Gemisch als mobile Phase.

Die Analysenergebnisse sind in Figur 1 zusammengefasst. Es wurde Rhaponticin im Blut detektiert mit einem Maximum bei 3-4 Stunden (Figur 1), während Rhapontigenin nicht gefunden werden konnte. Da Rhaponticin einer der Hauptinhaltsstoffe von ERr 731^{®} ist, ist davon auszugehen, dass Rhaponticin ein wirksamkeitsmitbestimmender Inhaltsstoff von ERr 731^{®} ist. Dies ist um so überraschender, als bisher davon ausgegangen wurde, dass nur die Aglycone, nicht jedoch die glycosylierten Hydroxystilbene wirksam sind (Park et al., Arch Pharm Res. 2002;25:528-533).

### b) vivo-Akkumulierung und -Metabolisierung der Inhaltsstoffe von ERr 731^{®} in Hundeplasma

1) 20 männlichen und 20 weiblichen Hunden (reinrassige Beagles, Gewicht 6-9 kg, Alter 6-8 Monate) wurden 100 (je 4 Tiere), 300 (je 4 Tiere) und 1000 (je 6 Tiere) mg/kg Körpergewicht/Tag ERr 731^{®} verabreicht. Am Tag 1 wurde den Tieren nach 0, 0.5, 1, 2, 4, 8 und 24 Stunden jeweils 5 ml Blut abgenommen und Plasma gewonnen. Aus diesem wurde, wie in Abschnitt a) beschrieben, die Analyse durchgeführt, um Rhaponticin, Desoxyrhaponticin, Rhapontigenin, Desoxyrhapontigenin, Resveratrol und Piceatannol im Blut nachzuweisen. Die Versuchsergebnisse sind in Figur 2a dargestellt.
2) Zur weiteren Aufklärung der Wirkungsweise wurde ERr 731^{®} drei männlichen und drei weiblichen Hunden (reinrassige Beagles, Gewicht 6-9 kg, Alter 6-8 Monate) in einer Dosis von 100 mg ERr 731^{®}/kg Körpergewicht oral per Kapsel verabreicht. Nach verschiedenen Zeiten wurde den Tieren Blut entnommen und Blutplasma gewonnen. Das Plasma wurde auf ERr 731^{®}-Inhaltsstoffe und -Metabolite untersucht. Überraschenderweise konnten sowohl in männlichen als auch in weiblichen Tieren signifikante Mengen des Metaboliten Piceatannol und geringe Mengen des Metaboliten Resveratrol nachgewiesen werden. Maximale Plasmaspiegel dieser Metabolite wurden nach etwa 24 h erreicht. Die Plasmaspiegel für Piceatannol lagen deutlich über denen von Resveratrol. Die Versuchsergebnisse zum Zeitpunkt 24 h sind in Figur 2b dargestellt.

Die oben beschriebenen Versuchsergebnisse belegen überraschenderweise, dass die Hauptinhaltsstoffe von ERr 731^{®} nach oraler Verabreichung als Glycoside im Körper resorbiert werden und als solche im Blutkreislauf in dosisabhängiger Weise nachweisbar und damit systemisch bioverfügbar sind, während deren direkte Aglycone Rhapontigenin und Desoxyrhapontigenin nicht nachweisbar waren. Außerdem konnte gezeigt werden, dass bei hoher Dosierung auch die korrespondierenden Metabolite Resveratrol und insbesondere Piceatannol im Körper gebildet werden.

### Testbeispiel 2: Östrogenrezeptor β (ERβ)-Aktivierung durch ERr 731^{®} und seinen Metaboliten in der ERβ-exprimierenden endometrialen Adenokarzinomzelllinie HEC-1 B

HEC-1B Zellen, eine humane endometriale Adenokarzinom-Zelllinie, exprimieren weder ERα noch ERβ. Damit stellen sie eine Möglichkeit dar, in einem humanen und endometrialen Kontext Liganden-abhängige Effekte von Substanzen auf die Transaktivierungsmechanismen hinsichtlich verschiedener Rezeptor-Subtypen und verschiedener östrogenresponsiver Promotoren zu untersuchen.

Eingesetzt wurde ein etabliertes ERβ/mC3-Luciferase-System. Dazu wurden HEC-1 B Zellen in 24-Well-Platten mit einer Dichte von 95.000 Zellen/Well ausgebracht (in Dul-becco's modified Essential Medium (DMEM/F12)). Am nächsten Tag wurden diese mit einem ERβ-enthaltenden Konstrukt (hERβ/pSG2) und einem triple-ERE-enthaltenden Promotor/Luciferase Konstrukt (mC3-Luc/pGL2) kotransfiziert (Hillisch et al. Dissecting physiological roles of estrogen receptor alpha and beta with potent selective ligands from structure-based design. Mol Endocrinol. 2004 Jul; 18:1599-609)Die transiente Transfektion erfolgte mittels DOTAP(N-[1-(2,3-Dioleoyloxy)]-N,N,N-trimethyl-ammonium-propan methyl-sulfat; Roth) wie vom Hersteller beschrieben.

Nach 24 Stunden wurden die Zellen mit entsprechenden Konzentrationen der zu untersuchenden Substanzen bzw. Substanzgemische behandelt. Als Positivkontrolle diente Estradiol (10 nM), als Lösungsmittelkontrolle wurde ein vergleichbares Volumen Dimethylsulfoxid (DMSO) eingesetzt. Die Inkubationszeit betrug 24 Stunden. Danach wurden die Zellen lysiert. Einerseits wurde mittels des Luciferase Assay^{®} Kits (Promega) die Luciferaseaktivität und andererseits mittels des BCA^{®} Kits (Sigma) der Proteingehalt bestimmt. Die resultierenden spezifischen Luciferase-Aktivitäten der zu untersuchenden Substanzen wurden dann gegen die DMSO-Kontrolle (100%) verglichen.

Für jede Prüfsubstanz werden mindestens drei Transfektionsexperimente durchgeführt. Nach Berechnung der relativen Luciferase-Aktivität in Bezug auf die Negativkontrolle (DMSO) jedes Einzelexperiments (gesetzt als 100%) werden die entsprechenden Mittelwerte und Standardabweichungen gebildet. Die Darstellung der Ergebnisse erfolgt grafisch in Form eines Säulendiagramms. Die Berechnung der Signifikanz erfolgt mittels Student's t-test, wobei diese wie folgt festgelegt wurde: * p < 0,05; ** p < 0,01; ***p<0,001.

Die Ergebnisse für ERr 731^{®} sind in Figur 3a, für trans-Rhapontigenin in Figur 3b und für Desoxyrhapontigenin in Figur 3c dargestellt. Sie zeigen, dass die Substanzen dosis-abhängig den ERß aktivieren und damit erfindungsgemäß eingesetzt werden können.

Die oben beschriebenen Versuchsergebnisse belegen den überraschenden Befund, dass, entgegen bisheriger Annahmen, die "Vorläufer" von Resveratrol und Piceatannol, d. h. die Glycoside Rhaponticin und Desoxyrhaponticin (als Hauptbestandteile von ERr 731®), per se von menschlichen Zellen aufgenommen werden können. Die Fachwelt ist bisher von einer Nichtresorbierbarkeit dieser Glykone ausgegangen (vgl. Park et al, Arch. Pharm. Res. 2002, 25 (4), 528-533). Weiterhin überrascht die Tatsache, dass die Wirkstoffkombination ERr 731^{®} höhere Wirksamkeit besitzt als die korrespondierenden Aglycone Rhapontigenin und Desoxyrhapontigenin, welche unter physiologischen Bedingungen jedoch nicht nachweisbar sind (vgl. Ergebnisse zu obigem Testbeispiel 1, Figur 2a).

Damit konnte durch vorliegende Erfindung die direkte pharmakologische Wirksamkeit der Glycoside Rhaponticin und Desoxyrhaponticin (als Hauptbestandteile von ERr 731®) erstmalig gezeigt werden.

### Testbeispiel 3: Wirkung von ERr 731^{®} auf IL-6

### a) Langzeit-Wirkung im Menschen

In vivo-Untersuchungen zu IL-6-Spiegeln im Blut erfolgten im Rahmen einer 15-monatigen Beobachtungsstudie, an der 82 Patientinnen mit klimakterischen Beschwerden beteiligt waren. Diese Patientinnen nahmen 1-mal täglich 1 Tablette ERr 731^{®} (Phytoestrol^{®} N; Dosierung = 4 mg ERr 731^{®}) ein. Vor der Einnahme (IC) und nach jeweils 3 Monaten wurde Blut abgenommen, und IL-6 mittels eines spezifischen ELI-SAs (Pharmingen BD, Heidelberg) im Serum detektiert.

Überraschenderweise wurde bei diesen Patientinnen nach einer 15-monatigen Behandlung (FA II) erstmals gefunden, dass mit ERr 731^{®} die IL-6-Spiegel signifikant gegenüber den Spiegeln vor der ersten Einnahme (IC) reduziert waren. Dieser überraschende Befund ist in beiliegender Figur 4 dargestellt.

### b) Untersuchung der Effekte von ERr 731^{®} auf die Zytokin-stimulierte Freisetzung von IL-6 aus humanen Tumorzellen

Für die Versuche wurde die humane Tumorzelllinie A549 (Lungenkarzinomzellen) verwendet.

Diese Zellen stellen ein Modellsystem für IL-6-produzierende Zellen bei entzündlichen Erkrankungen dar (Billich et al., Basal and induced sphingosine kinase 1 activity in A549 carcinoma cells: function in cell survival and IL-1β and TNF-α induced production of inflammatory mediators. Cell Signal 2005; 17: 1203-1217).

A549 Zellen sind menschliche Lungenkarzinomzellen (58-jähriger männlicher Patient, 1972), die die Fähigkeit besitzen, in geeigneten Mausmodellen Tumore bilden zu können. Diese epithelialen Zellen sind ein weit verbreitetes Zellkulturmodell zur pharmakologischen Beeinflussung von Karzinomzellen der Lunge.

A549 Zellen wachsen adherent, haben eine Generationszeit von circa 30 h und werden in FCS-haltigen (10%) DMEM Zellkulturmedium kultiviert. Stimuliert wurde mit einer Kombination folgender rekombinanter humaner Zytokine:
IL-1β (50 ng/ml)
TNFα (50 ng/ml).

Zur Stimulation wurden konfluente A549 Zellen (in "6-well Plates") in DMEM Stimulationsmedium (ohne Phenolrot, serumfrei, in 0.01% fettsäurefreiem BSA) mit IL-1β/TNFα +/- ERr 731^{®} aktiviert.

Die jeweiligen Extraktkonzentrationen (Stock: 10 mg/ml in DMSO; getestete Konzentrationen: 0.1 ng/ml bis 10 µg/ml), sind der Figur 5 zu entnehmen. Im Rahmen der Stimulationen wurde in allen Kulturansätzen diejenige DMSO Konzentration erzeugt, die sich aus der höchsten ERr 731^{®}-Endkonzentration der jeweiligen Versuchsserie ergab (0.1 % DMSO bei 10 µg/ml, 0.01% DMSO bei 1 µg/ml).

Nach einer 24-stündigen Inkubation wurden die Kulturüberstände abzentrifugiert und die zugehörigen IL-6 Konzentrationen in den zellfreien Überständen (je drei Schalen pro Bedingung) in Doppelbestimmung mittels eines spezifischen ELISAs für humanes IL-6 gemessen.

Die Kombination IL-1β/TNFα führte in allen Versuchsserien zu einer robusten Induktion von IL-6. Die Stimulierbarkeit der A549 Zellen durch IL-1β/TNFa schwankte an einzelnen Versuchstagen zwischen 1000 pg/ml und 5000 pg/ml. Die Effekte von ERr 731^{®} zeigten in den Versuchsserien keine Korrelation zur Stärke der Ausgangsstimulation.

Ein repräsentatives Versuchsergebnis ist in beiliegender Figur 5 dargestellt:
Man erkennt eine etwa 28%ige Hemmung der IL-6 Freisetzung bei allen ERr 731^{®} Konzentrationen. Damit konnte überraschenderweise erstmalig gezeigt werden, dass ERr 731^{®}, d.h. dessen Inhaltsstoffe Rhaponticin und Rhapontigenin, eine partielle Reduktion der IL-6 Freisetzung aus A549 Zellen bewirken.

Der beobachtete Effekt auf die IL-6-Produktion ist überraschend, da bisher davon ausgegangen wurde, dass glycosidische Hydroxystilbene, wie sie auch in der erfindungsgemäßen Wirkstoffkombination enthalten sind, keinen Effekt auf die Mediatorproduktion in diesen Zellen haben (Donelly et al. Anti-inflammatory effects of resveratrol in lung epithelial cells: molecular mechanisms. Am J Physiol Lung Cell Mol Physiol 2004; 287: L774-L783).

Dies ist insbesondere wichtig für die Therapie Östrogen-unabhängiger Tumore (Mamma-, Prostatakarzinom).

### Testbeispiel 4: Östrogene Aktivität von ERr 731^{®}in ERα-exprimierenden Zellsystemen

Es sollte die Frage geklärt werden, ob ERr 731^{®} zusätzlich zu ERß auch den ERα aktiviert, oder spezifisch für ERß ist. Deshalb wurde der Extrakt ERr 731^{®} im Vergleich mit Östradiol auf die östrogene Aktivität in etablierten Modellsystemen untersucht.

### Test A:

In einer ersten Serie von Experimenten wurde ein rekombinanter Hefe-Screen verwendet (vgl. E. Routledge and J.P. Sumpter, Östrogenic activity of surfactants and some of their degradation products assessed using a recombinant yeast screen, Envirom. Tox. Chem.1996).

*Saccharomyces-cerevisiae-*Zellen wurden mit humanem ER a sowie mit einem Reporter-Gen, bestehend aus dem jeweiligen responsiven Promotorelement fusioniert mit dem LacZ-Gen, welches für β-Galaktosidase kodiert, stabil transfiziert. Durch Östrogenbehandlung (mit Östrogen oder einem östrogenähnlich wirkenden Substrat) der Zellen wird, vermittelt durch den Östrogenrezeptor, β-Galaktosidase aktiviert, was zu einer Rotfärbung der Hefezellen führt, welche bei 565 nm gemessen werden kann. Die Versuchsergebnisse sind in Figur 6a zusammengefasst.

### Test B:

In einer zweiten Serie von Experimenten wurden die Daten der Östrogenizitätsmessung durch Bestimmung der Induktion von alkalischer Phosphatase in Ishikawa-Zellen (menschliche endometriale Adenocarcinomzellen) verifiziert, welche mit einem ER α - enthaltenden Reportergenkonstrukt transfiziert worden waren. Die Aktivität von alkalischer Phosphatase, welche über das chromogene Substrat 4-Nitrophenylphosphat bewertet wird, stellt eine ER a -vermittelte Antwort dar.

Der Test basiert auf der Beschreibung von Holinka CF, Hata H, Kuramoto H, Gurpide E (1986) Effects of steroid hormones and antisteroids on alkaline phosphatase activity in human endometrial cancer cells (Ishikawa Line). Cancer Res. 46: 2771-2774, sowie Modifikationen gemäß Wober J, Weißwange I, Vollmer G (2002) Stimulation of alkaline phosphatase activity in Ishikawa cells induced by various phytoestrogens and synthetic estrogens. J. Steroid Biochem. Mol. Biol. 83:227-233.

Tabelle 2 stellt die Konzentrationen für die positive Kontrolle (Östradiol) und die Testsubstanzen dar, welche in dem Assay verwendet wurden.

**Tabelle 2**

| **Testsubstanz** | **Konzentration (M)¹** |
|---|---|
| Östradiol | 10⁻⁶ |
| Resveratrol | 10⁻⁸-10⁻⁵ |
| trans-Rhapontigenin | 10⁻⁸-10⁻⁵ |
| Desoxyrhapontigenin | 10⁻⁸-10⁻⁵ |
| Piceatannol | 10⁻⁷-10⁻⁵ |
| cis-Rhapontigenin | 10⁻⁸-10⁻⁵ |
| Extrakt ERr 731^{®} | 0,00001-10 |

| | |
|---|---|
| ¹Ausnahme: Die Konzentration für den Extrakt ERr 731^{®} ist in µg/ml angegeben | |

Die Ergebnisse zu Test B sind in Figur 6b für ERr 731^{®} dargestellt.

Beide Tests sind stichhaltig, da sämtliche positive und negative Kontrollen ihre vorausgesagten Effekte (vgl. Figuren 6a und b) zeigen. Die Ergebnisse beider oben beschriebener Testsysteme belegen, dass weder ERr 731^{®} noch dessen Inhaltsstoffe und Metabolite in den hier verwendeten Zellsystemen einen signifikanten Einfluss auf die Aktivierung von ERα aufweisen. Somit kann angenommen werden, dass die Aktivität von ERr 731^{®} insbesondere in Endometriumzellen auf einem von ERα unabhängigen molekularen Mechanismus basiert.

Zusammenfassend ist festzustellen, dass die erfindungsgemäße Wirkstoffzusammensetzung in Endometriumzellen keine unerwünschte ERα-aktivierende Wirkung besitzt, sondern vielmehr spezifisch ERß aktiviert, (vgl. Testbeispiel 2, oben).

### Testbeispiel 5: Anti-androgene Aktivität der Inhaltsstoffe und Metabolite von ERr 731^{®} in einem Androgenrezeptor-exprimierenden Hefezellsystem

In einer Serie von Experimenten wurde ein rekombinanter Hefescreen (E. Routledge and J.P. Sumpter, Östrogenic activity of surfactants and some of their degradation products assessed using a recombinant yeast screen, Envirom. Tox. Chem.1996*.)* verwendet. Die *Saccharomyces-cerevisiae-*Zellen wurden mit humanem Androgenrezeptor sowie mit einem Reporter-Gen, bestehend aus dem jeweiligen Response-Element fusioniert mit dem LacZ-Gen, welches für β-Galaktosidase kodiert, stabil transfiziert.

Als erstes wurde eine aufgetaute Hefe-Stammkultur als Übernachtkultur in 52 ml Wachstumsmedium bei 28-32°C für 24h unter Schütteln angezogen. Am nächsten Morgen wurden für die Testkultur 0,5ml der Übemachtkultur mit einer optischen Dichte (OD) von 1 entnommen und mit neuen 52 ml Wachstumsmedium plus CPRG-Enzymsubstrat gegeben. In die Wells der 96-Mikrotiterplatten wurden jeweils 1-2 µl der in Ethanol oder DMSO gelösten Substanz (4-facher Ansatz) pipettiert. Als Standardsubstanz diente Dihydrotestosteron (DHT). Dann wurde das Gemisch von Hefen und Testmedium vorsichtig aufgeschüttelt und je Well 200µl der Testkultur dazu pipettiert und bei 32°C 2-3 Tage inkubiert. Nach der 2-3tägigen Inkubation wurde die Färbung bei 565 nm und die Trübung bei 690 nm gemessen.

Die androgene bzw. anti-androgene Aktivität wurde über die Bestimmung von β-Galaktosidase gemessen, die zu einer Rotfärbung der Hefezellen führt, welche bei 565 nm gemessen werden kann. In Tabelle 3 sind die Konzentrationen für die positive Kontrolle (DHT) und die Testsubstanzen, welche in dem Assay verwendet wurden, angegeben.

**Tabelle 3**

| **Testsubstanz** | **Konzentration (M)** |
|---|---|
| DHT | 10⁻¹²-10⁻⁶ |
| Desoxyrhapontigenin | 10⁻¹¹-10⁻⁵ |
| Piceatannol | 10⁻¹⁰-10⁻⁵ |

Erfindungsgemäß wurde in diesen Experimenten festgestellt, dass in diesem Zellkultursystem nicht nur bestimmte Metabolite (Piceatannol) sondern auch Inhaltsstoffe (Desoxyrhapontigenin) von ERr 731^{®} anti-androgene Effekte durch Prävention der Bindung von DHT an den Androgenrezeptor in einem Konzentrationsbereich von 1-10 µM aufweisen (Figur 7A, B).

Die Aussagekraft dieses Tests wurde unter Verwendung des kompetitiven Androgenrezeptorantagonisten Hydroxyflutamid als positive Kontrolle belegt (Figur 7 C).

Die Versuchsergebnisse weisen darauf hin, dass insbesondere auch Inhaltsstoffe von ERr 731^{®} mit verschiedenen Androgenrezeptor-vermittelten Signalwegen, welche zu Prostatakrebs und LUTS führen können, interagieren.

In einer zweiten Serie von Experimenten in diesem Zellkultursystem (Versuche nicht gezeigt) wurde festgestellt, dass weder einzelne Inhaltsstoffe noch die oben genannten Metabolite von ERr 731^{®} noch der Gesamtextrakt ERr 731^{®} selbst den Androgenrezeptor aktivieren. Dies bedeutet, dass ERr 731^{®} und seine Inhaltsstoffe bzw. Metabolite keinen androgenen Effekt aufweisen.

## Patentansprüche

1. Verwendung einer Hydroxystilben-haltigen Wirkstoffkombination wobei die Wirkstoffkombination im Wesentlichen Rhaponticin und Desoxyrhaponticin umfasst, zur Herstellung eines Mittels zur Behandlung von Prostatakrebs und/oder von Lower Urinary Tract Symptoms (LUTS), wobei Rhaponticin und Desoxyrhaponticin in einem Gewichtsverhältnis von etwa 10:1 bis 1:10 enthalten sind.

2. Verwendung nach Anspruch 1, wobei die Wirkstoffkombination a) einen Hydroxystilben-Gesamtgehalt von mehr als 90 Gew.-%; und/oder b) einem Aglykon-Anteil von weniger als 5 Gew.-%; und/oder c) einen Gehalt von weniger als 0,5 Gew.-% an Anthrachinon und/oder Anthrachinoiden aufweist.

3. Verwendung nach einem der vorhergehenden Ansprüche, wobei die Hydroxystilben-Komponenten der Wirkstoffkombination chemisch synthetisiert oder aus Pflanzen isolierbar ist.

4. Verwendung nach einem der vorhergehenden Ansprüche, wobei die Wirkstoffkombination Rhaponticin, Desoxyrhaponticin sowie Rhapontigenin und/oder Desoxyrhapontigenin umfasst.

5. Verwendung nach einem der vorhergehenden Ansprüche, wobei die Wirkstoffkombination im wesentlichen aus etwa
60-70 Gew.-% Rhaponticin
30-40 Gew.-% Desoxyrhaponticin
0-2 Gew.-% trans-Rhapontigenin
0-2 Gew.-% Desoxyrhapontigenin
besteht.

6. Verwendung nach einem der vorhergehenden Ansprüche, wobei der Wirkstoff oder die Wirkstoffkombination erhältlich ist aus Pflanzen der Gattung Rheum spp, Astragalus spp, Cassia spp oder Picea spp.

7. Verwendung nach einem der vorhergehenden Ansprüche, wobei der Wirkstoff oder die Wirkstoffkombination erhältlich ist aus Wurzeln und/oder andere Pflanzenteilen von Rheum rhaponticum.

8. Verwendung nach einem der vorhergehenden Ansprüche, in Kombination mit wenigstens einem weiteren zur Prävention und/oder Behandlung einer in Anspruch 1 definierten Erkrankung geeigneten und von Verbindungen gemäß der Definition in Anspruch 1 verschiedenen Wirkstoff.

9. Verwendung nach einem der vorhergehenden Ansprüche, wobei das Mittel ausgewählt ist unter chemisch-synthetischen, biotechnologisch hergestellten, pflanzlichen und homöopathischen Arzneimitteln, anderen Arzneipflanzenzubereitungen, Nahrungsergänzungsmitteln und diätetischen Lebensmitteln.

10. Hydroxystilben-haltige Wirkstoffkombination, welche wie in einem der Ansprüche 1 bis 8 definiert ist, zur Verwendung bei der Behandlung von Prostatakrebs und/oder LUTS.

## Claims

1. Use of a hydroxystilbene-containing active ingredient combination, where the active ingredient combination comprises essentially rhaponticin and desoxyrhaponticin for producing a composition for treading prostate cancer and/or Lower Urinary Tract Symptoms (LUTS), where rhaponticin and desoxyrhaponticin are present in a weight ration of about 10:1 to 1:10.

2. Use according to Claim 1, where the active ingredient combination has a) a Hydroxystilbene total content of more than 90% by weight; and/or b) an aglycone fraction of less than 5% by weight; and/or c) a content of less than 0.5% by weight of anthraquinone and/or anthraquinoids.

3. Use according to one of the preceding claims, where the hydroxystilbene components of the active ingredient combination are chemically synthesized or can be isolated from plants.

4. Use according to one of the preceding claims, where the active ingredient combination comprises rhaponticin, desoxyrhaponticin and rhapontigenin and/or desoxyrhapontigenin.

5. Use according to one of the preceding claims, where the active ingredient combination consists essentially of about
60-70% by weight of rhaponticin
30-40% by weight of desoxyrhaponticin
0-2% by weight of trans-rhapontigenin
0-2% by weight of desoxyrhapontigenin.

6. Use according to one of the preceding claims, where the active ingredient or the active ingredient combination is obtainable from plants of the genus Rheum spp, Astragalus spp, Cassia spp or Picea spp.

7. Use according to one of the preceding claims, where the active ingredient or the active ingredient combination is obtainable from roots and/or other plant parts of Rheum rhaponticum.

8. Use according to one of the preceding claims, in combination with at least one further active ingredient that is suitable for the prevention and/or treatment of a disease defined in Claim 1 and which is different from compounds according to the definition in Claim 1.

9. Use according to one of the preceding claims, where the composition is selected from chemosynthetic, biotechnologically produced, plant and homeopathic medicaments, other medicinal plant preparations, food supplements and dietetic foods.

10. Hydroxystilbene-containing active ingredient combination which is defined as in one of Claims 1 to 8, for use in the treatment of prostate cancer and/or LUTS.

## Revendications

1. Utilisation d'une association de substances actives contentant des hydroxystilbènes, l'association de substances actives comprenant essentiellement de la rhaponticine et de la désoxyrhaponticine, pour la fabrication d'une composition destinée au traitement du cancer de la prostate et/ou de symptômes des voies urinaires inférieures (SVUI), la rhaponticine et la désoxyrhaponticine étant contenues en un rapport pondéral d'environ 10:1 à 1:10.

2. Utilisation selon la revendication 1, dans laquelle l'association de substances actives présente a) une teneur en hydroxystilbènes de plus de 90 % en poids ; et/ou b) une proportion d'aglyccnes de moins de 5 % en poids ; et/ou c) une teneur de moins de 0,5 % en poids en anthraquinone et/ou anthraquinoïdes.

3. Utilisation selon l'une quelconque des revendications précédentes, dans laquelle les composants hydroxystilbène de l'association de substances actives sont synthétisés chimiquement ou isolables à partir de plantes.

4. Utilisation selon l'une quelconque des revendications précédentes, dans laquelle l'association de substances actives comprend de la rhaponticine, de la désoxyrhaponticine ainsi que la rhapontigénine et/ou de la désoxyrhapontigénine.

5. Utilisation selon l'une quelconque des revendications précédentes, dans laquelle l'association de substances actives est essentiellement constituée d'environ
60-70 % en poids de rhaponticine
30-40 % en poids de désoxyrhapontîcine
0-2 % en poids de trans-rhapontigénine
0-2 % en poids de désoxyrhapontigénine.

6. Utilisation selon l'une quelconque des revendications précédentes, dans laquelle la substance active ou l'association de substances activés peut/peuvent être obtenue(s) à partir de plantes appartenant au genre *Rheum spp., Astragalus spp., Cassia spp* ou *Picea spp.*

7. Utilisation selon l'une quelconque des revendications précédentes, dans laquelle la substance active ou l'association de substances actives peut/peuvent être obtenue(s) à partir de racinés et/ou d'autres parties de plantes de *Rheum rhaponticum.*

8. Utilisation selon l'une quelconque des revendications précédentes, en association avec a moins une autre substance active différente des composés selon la définition dans la revendication 1 et appropriée à la prétention et/ou au traitement d'une maladie définie dans la revendication 1.

9. Utilisation selon l'une quelconque des revendications précédentes, dans laquelle la composition est choisie parmi des médicaments produits par synthèse chimique, par biotechnologie, végétaux et homéopathiques, d'autres préparations de plantes médicinales, des compléments nutritionnels et des aliments diététiques.

10. Association de substances actives contenant des hydroxystilbènes, qui est telle que définie dans l'une quelconque des revendications 1 à 8, pour utilisation dans le traitement du cancer de la prostate et/ou d'un SVUI.
